# EUROPEAN PATENT SPECIFICATION

(11) **EP 1 209 186 B1**
(45) Date of publication and mention of the grant of the patent: **02.08.2006**
(21) Application number: 00929871.2
(22) Date of filing: 25.05.2000
(51) Int. Cl.: C08J 5/02, C08J 7/12, C08J 3/24, C08C 19/42, A61F 6/04, A61F 13/10, A41D 19/04, B29C 41/14, B05D 3/10

(54) **NONTACKY LATEX PRODUCTS**
NICHTKLEBRIGE LATEXGEGENSTÄNDE
PRODUITS DE LATEX NON COLLANTS

(30) Priority: 28.05.1999 JP 14971899; 18.03.2000 JP 2000121767
(43) Date of publication of application: 29.05.2002
(73) Proprietor: Suzuki Latex Industry Co., Ltd., Chiba 261-0023 (JP)
(72) Inventor: KOIDE, Kazuo, Yotsukaido-shi, Chiba 284-0045 (JP); SUZUKI, Takayuki, Sakura-shi, Chiba 285-0831 (JP); SUZUKI, Takahisa, Funabashi-shi, Chiba 273-0021 (JP)
(74) Representative: HOFFMANN EITLE
(86) International application number: PCT/JP2000/003370
(87) International publication number: WO 2000/073367

(56) References cited:
- EP-A- 0 486 183
- EP-A- 0 524 836
- EP-A1- 0 486 183
- EP-A1- 0 921 133
- BE-A- 547 273
- DE-A- 1 906 901
- FR-A- 1 149 536
- GB-A- 785 631
- GB-A- 1 220 384
- GB-A- 1 365 253
- GB-A- 1 480 112
- GB-A- 2 137 627
- JP-A- 5 186 902
- JP-A- 6 048 066
- JP-A- 6 340 758
- JP-A- 8 072 170
- JP-A- 59 199 701
- JP-Y2- 7 019 819
- US-A- 2 937 164
- US-A- 3 740 357
- US-A- 3 743 612
- US-A- 3 753 941
- US-A- 3 767 605
- US-A- 5 195 537
- ZAKHAROV N D: "VULCANIZATION OF CARBOXYLIC RUBBERS" RUBBER CHEMISTRY AND TECHNOLOGY, RUBBER DIVISION ACS. AKRON, US, vol. 36, no. 3, 1963, pages 568-574, XP001109665 ISSN: 0035-9475
- BROWN H P ET AL: "CROSSLINKING REACTIONS OF CARBOXYLIC ELASTOMERS" RUBBER CHEMISTRY AND TECHNOLOGY, RUBBER DIVISION ACS. AKRON, US, vol. 36, no. 4, 1963, pages 931-962, XP001109664 ISSN: 0035-9475

## Description

### [Technical Field]

The present invention relates to a novel non-adhesive carboxylated latex product. Further, the present invention provides a latex product with high durability on wear.

### [Background Art]

Carboxylated latex products - for example, dipping products such as balloons, gloves, fingerstalls and condoms; extruded products such as rubber threads and rubber pipes; cast- molded products such as balloons and toys; and whole rubber products and products having rubber surface such as rubber sheets, hoses and draw cloths - are apt to have adhesive surfaces to result in damaging the processability of the products or producing defective articles. Further, they have the defect in that products having desirable shapes cannot be fabricated.

Heretofore, to overcome these defects, adhesion preventing agents have been used, and as the adhesion preventing agents powdered substances are normally used, which are called dusting powder (or simply powder). As the powder, generally used are mica, talc, calcium carbonate, white carbon and corn starch.

The powder, however, spreads to an article in contact with the latex product and becomes a cause of various troubles if the article belongs to the field of precision instrument. Also, appearance of the latex product is damaged. In July 1999, FDA issued a regulation on medical rubber gloves that the powder quantity on synthetic rubber medical gloves should be controlled 120 mg or less per piece of gloves. On the other hand, as to natural rubber medical gloves, the regulation was issued that controlled the soluble protein to 1200 *µ*g per piece of gloves to prevent the latex allergy derived from protein. And, a shift from natural rubber gloves to synthetic rubber gloves is anticipated. Thus, making the synthetic rubber products non-adhesive is an important technical issue of the field.

The general method of adhesion prevention other than the powder includes halogenation by post-chlorination treatment. For example, USP 3,411,982 and 3,740,262 disclose that halogenation makes the surface of rubber gloves slippery. USP 4,304,008 discloses halogenation, in place of the powder, facilitates wearing rubber products. USP 3,740,262 discloses halogenating gloves to provide the outside surface without the powder and the inside surface with the powder.

The halogenation is a method in which a thin layer of halogenated rubber is made on the product surface to prevent adhesion and blooming, and this method is fairly widely conducted, providing a rubber product having a clean surface without the powder. However, when the degree of halogenation is too high, discoloration occurs, the surface becomes brittle to cause cracks, or heat resistance is deteriorated. Further, it is feared for the contacting metal to be corroded. Above all things, many users hesitate to use such products because of their being environmentally undesirable.

USP 4,304,008 discloses surgical gloves wherein the inside layer comprises a natural rubber and the outside layer a halogen-resistant silicone, the inside layer being halogenated to give a non-adhesive property.

The defect of halogenation is admitted by USP 5, 284, 607 . In the patent, medical gloves are formed using an acid-soluble powder, which is then dissolved by treating with an acid such as nitric acid, and thereafter the gloves are chlorinated by a bleaching agent.

There are found a variety of improvements in the methods of manufacturing rubber products which use powders or substances having particulate structures.

USP 4,070,713 discloses medical gloves comprising two layers, i.e., outside and inside layers, of elastic substances. In the inside layer a particulate substance such as zinc oxide on titanium oxide is firmly embedded, and moreover on the inside surface to come in contact with the skin, the particles are exposed partially.

USP 4,143,109 discloses a manufacture method relating to the above-mentioned patent.

USP 5,138,719 discloses a method for making powder-free gloves, fingerstalls and the like from latex and microcapsule. The microcapsule is dispersed and distributed in the latex so that its concentration may increase from the outside surface of the product toward the inside surface thereof to build a concentration gradient. The microcapsule concentration is high on the inside surface, and this gives a good slipping property and an easy wearing without the powder.

USP 5,881,386 discloses gloves with the two layers comprising polyvinylchloride and polyesterpolyurethane. The inside polyesterpolyurethane layer is incorporated with a particulate substance of 1 to 75 *µ*m.

Japanese Patent Application Laid-Open (Kokai) No.11-12823 discloses, regarding the working gloves for clean room manufactured from polyvinylidene chloride paste sol, a technology of manufacturing working gloves with improved dusting properties by dipping them in an inside surface treating agent containing a powder of 0.1 to 1.5 *µ*m followed by drying.

Japanese Patent Application Laid-Open (Kokai) No.11-61527 discloses rubber gloves excellent in wearing and removing which are provided with a slippery resin layer by dipping in an aqueous dispersing liquid containing a synthetic rubber latex that is not coagulated by a coagulant contained in gloves themselves and containing an organic filler.

Japanese Patent Application Laid-Open (Kohyo) No.9-501983 discloses a water-dispersable silicone modified dispersion powder composition and the method of manufacturing it, and describes that the composition can be used as antiblocking agent.

In recent years, such products have come to be developed that various substances are coated on the surface of latex products.

USP 4,310,928 provides surgery gloves by dispersing an oil and fat or lipophilic substance in a coagulation liquid and coating it on the surface of a natural rubber. Here, to prevent separation of the oil and fat or lipophilic substance, a surface active agent is added in the coagulation liquid.

USP 5,780,112 and 5,974,589 disclose a method of attaching a high density straight-chain hydrocarbon polymer, especially polyethylene, to the surface of a natural rubber by chlorine generated by acidifying a hypochlorite, and the latex product thus treated is non-adhesive without the powder.

Japanese Patent Application Laid-Open (Kohyo) No.11-507085 discloses a flexible copolymer coating which can be bonded firmly to the surface of a rubber article and extend without separation from the bonded surface. The patent, taking into consideration the removal property from the dipping former and the dry and wet wearing property, discloses an emulsion type copolymer between a reactive, low surface energy monomer, preferably a silicone oligomer, and an alkyl acrylate and a reactive hard monomer.

There are many disclosures on the method for preparing the powder free gloves by coating a hydrophilic hydrogel forming polymer on the rubber surface and curing the polymer layer. The examples thereof are USP 3,326,742; 3,585,103; 3,607,473; 3,745,042; 3,901,755; 3,925,138; 3,930,076; 3,940,533; 3,966,530; 4,024,317; 4,110,495; and 4,125,477.

Further, USP 4,499,154 discloses a method of manufacturing a talc-free product by immersing a dipping former in a natural rubber latex, leaching the product in hot water, impregnating the product with a dilute acid, neutralizing the surface with water or an aqueous alkali solution, dipping in a hydrophilic hydrogel formable polymer such as a copolymer of 2-hydroxyethylmethacrylate and methacrylic acid or 2-ethylhexylacrylate and a crosslinking agent thereof, heating the coat layer to fix to the rubber, vulcanizing the rubber, removing the product from the dipping former, coating a silicone containing a surfactant, and providing heat. This method also discloses that the slipping property for wet hands is improved by crosslinking the coat layer of the hydrogel polymer of the invention and thereafter by treating with a cationic surfactant such as a long chain aliphatic amine. The patent also discloses that impregnating the rubber surface with an aluminum salt after acid treatment is preferable. This method can afford powder-free rubber products, but it includes many steps and raises the manufacturing costs excessively and it cannot be adopted for the product which dislikes the contamination of silicone.

USP 4,575,476 discloses that the coat layer of a specific 2-oxyethyl methacrylate hydrogel polymer has a good slipping property for dry hands. Further, by treating the above-mentioned hydrogel coat layer with a surfactant, specially with a cationic surfactant and a long chain aliphatic amine, the slipping property for wet hands is improved. Furthermore, by treating with a silicone containing surfactant, the adhesiveness of the surface without the hydrogel coat is markedly improved.

USP 5,688,855 discloses that the hydrophilicity of a solid surface gives lubrication to the surface in the presence of water, and that a hydrophilic concentration gradient is automatically generated within the coat layer by dissolving in one solvent a hydrogel forming polymer component and a water-soluble polymer component that has a low compatibility with the above component, coating them on the surface of a rubber product, and evaporating the solvent to cause phase separation of the two components.

Japanese Patent Application Laid-Open (Kokai) No.11-269708 discloses gloves laminated with a lubricant layer of a rubber or resin containing collagen on the inside surface of gloves provided with a base layer of a rubber or polymer.

The defect of coating on the rubber surface is that layer separation occurs when the rubber is extended.

USP 4,499,154 discloses reinforcing the adhesion of the coat layer by undercoating an acid on the rubber surface.

WO93/06996-A1 proposes using as a coat layer a polymer having a repeated structure of specific ether group and ester group.

USP 4, 548, 844 discloses an improvement of adhesion between a rubber layer and a hydrogel layer through acid treatment and also discloses that, by undercoating an aluminum cation or a cation of trivalence or more prior to the coating of the hydrogel polymer or by adding it to the hydrogel polymer, the adhesion of the rubber layer and the hydrogel polymer is enhanced. It is presumed that this is caused by bonding of a hydroxy or carboxyl group in the hydrogel polymer to a protein in the rubber latex.

Japanese Patent Application Laid-Open (Kokai) No.6-70942 discloses a multilayer product comprising a first layer formed from a natural rubber, a second layer of a natural rubber, polyurethane, poly(acrylamide/acrylic acid) and polyethylene oxide, and a third layer of an acryl copolymer and fluorocarbon telomer resin. The product is wearable under dry or wet conditions without the powder.

Japanese Patent Application Laid-Open (Kokai) No.10-95867 discloses production of powder-free medical gloves by coating a lubricating composition comprising the first and second ingredients on the surface of an elastomer product on the wearer's side with which the elastomer product is contacted. Here, the first composition comprises at least one compound selected from the group consisting of acetylene diol, organosilicone, amino modified silicone, and cationic surfactant. The second composition is at least one compound selected from the group consisting of cationic surface active agent, organosilicone, amino-modified silicone and acetylene diol.

Japanese Patent Publication (Kokoku) No.7-4405 discloses a method of treating a surface with a modified polysiloxane.

The methods for manufacturing rubber products containing no dusting powder includes the method in which a bivalent coagulant metal salt such as calcium nitrate and a latex stabilized by adding a water soluble surfactant stable against the said metal salt, preferably a nonionic surfactant, or a resin polymer are brought into co-existence in a coagulation liquid, and the former coated with the said coagulant is dipped in the latex to coat the one side of the rubber product. Although this method fails to make the rubber product substantially non-adhesive, it is possible to make the rubber product non-adhesive by adding a peeling or anti-adhesion agent to such coagulant composition as the third component.

USP 3,286,011 and 3,411,982 to Kavalir et al. disclose such technology above mentioned. The patent, however, fails to make the product powder-free because it adopts the powder as the peeling agent.
The patent discloses that polyvalent metal salts like calcium, magnesium and aluminum can be used as the latex coagulants.

The above-mentioned USP 4,310,928 discloses a method of fabricating surgery gloves which can be removed from a dipping former by dispersing a lipophilic substance into a coagulant liquid such as calcium nitrate by using such a coagulant.

Japanese Patent Application Laid-Open (Kohyo) No.10-508899 discloses a method for producing a rubber article freed from the powder by adding to the coagulant, an acrylic emulsion copolymer coating composition and a silicone emulsion. The said coating composition is made by copolymerization of a reactive silicone acrylate, an alkyl acrylate and a hard monomer, but such a composition is a known substance. The patent clarifies that the release becomes easy only when the silicone emulsion is added to the composition and that the gloves shows excellent dry-type and wet-type wearing performance.

EP 640,623 discloses a coagulant for natural rubber comprising a salt-stable polychloroprene or polyurethane and a bivalent metal salt and to that powder-free rubber gloves can be manufactured by further adding in the coagulant a peeling agent consisting of a polyethylene wax emulsion and a cationic surfactant.

In Japanese Patent Application Laid-Open (Kokai) No.11-236466 surfactants and various waxes such as polypropylene wax emulsion in place of the polyethylene wax emulsion were employed as adhesion eliminating agent or releasing agent, wherein the cationic surfactant functions for stabilizing the polychloroprene added to the coagulant and functions as a release agent between the polychloroprene and the dipping former because the affinity for the dipping former is higher than that for the pertinent polymer.

Japanese Patent Publication (Kokoku) No.2-42082 discloses a coagulant composition formed by adding a latex, a surfactant or bivalent or trivalent metal salt into water.

In Japanese Patent Application Laid-Open (Kohyo) No.9-511708 the Teague method is adopted to the manufacture of polyurethane coated gloves. Namely, a first layer is formed by dipping in an aqueous dispersion liquid or emulsion of a polyurethane polymer or copolymer, and the first layer is dipped in a coagulant then a latex compound to form a second layer. This literature also discloses a method of forming a lubricating polymer layer on the second layer.

There is disclosed technology regarding to powder-free gloves which use novel raw materials.

USP 5,851,683 proposes a special successive copolymerization polymer as to powder-free gloves comprising a thermoplastic elastomer for clean room.

As mentioned above, the method of preventing the adhesion of emulsion /latex products is important in both sides of manufacture and use of the products. Although various proposals have appeared, many of such technologies are considerably complicated and such a method that is simple, effective and economical has not been developed. One reason for this is that, since the main material is a natural rubber latex, a natural substance having strong adhesion and complicated causes for the adhesion, the treatment materials and techniques for the adhesion prevention possibly become complex.

The present invention, taking into consideration the above-mentioned current status of adhesion treating technology, is to provide a novel powder-free, non-adhesive carboxylated latex product. Further, the present invention provides a latex product having excellent durability in wear without sulfur vulcanization.

### [Disclosure of the Invention]

We studied intensively to solve the above-mentioned problems, and how found that the adhesion of the surface is strong if the surface of a carboxxylated latex product is not sufficiently crosslinked with a bivalent metal compound as a coagulant and further that, even if sufficient crosslinking is conducted with a mono- or bi-valent metal compound, non-adhesion is insufficient since heating causes the adhesion between the inner and outer surfaces of the latex product. Assuming that the cause of the adhesion of a carboxylated latex product may lie in that the carboxyl group of the latex forms a hydrogen bond through water, we studied methods to inhibit this hydrogen bond formation.

We first studied use of a metal compound having tri- or more valence as an external crosslinking agent. For example, an aluminum salt which is a representative trivalent metal cation possesses very strong coagulating ability according to the Shultz-Hardy Law , but when only aluminum compound is used as the external coagulant for the purpose of making a dipped product only the extremely thin film is formed. However, we found that a dip film having a usual thickness is formed by forming a layer of an external crossliking agent of aluminum compound on a dipping former followed by forming thereon a coagulant layer of a usually used mono- or bi-valent metal salt then dipping into the latex solution and further found that aluminum compound crosslinking layer is formed in the innermost layer. Further surprisingly, it was found that the inside surface of the dip film crosslinked with the aluminum compound is non-adhesive and that bringing each of the inside surfaces into close contact followed by heating under wet conditions does not cause the adhesion of each.

On the other hand, when the external crosslinking agent layer of the aluminum compound is formed on the external coagulant layer of mono- or bi-valent metal salt and subsequently dipped into the latex solution, only a thin film is formed. However, when external crosslinking agent layer is formed on the external coagulant layer comprises a mixture of the mono- or bi-valent metal salt and the aluminum compound followed by dipping in the latex solution, surprisingly a film having usual thickness and non-adhesive inside surface is formed.

Next, we studied on making the outside surface of the dip film non-adhesive. A film was shaped by forming the external coagulant layer on the dipping former and dipping it into the latex. The film was further dipped into the aluminum compound solution according to the usual method, followed by heating, thereby forming any aluminum compound crosslinking layer on the outside surface of the film. The outside surface of the film was non-adhesive. Further, after releasing the dip product from the dipping former, the both surfaces were dipped in the aluminum compound solution and subjected to subsequent heating. The both surfaces were found non-adhesive.

Based on the above-mentioned findings, the crosslinking layer formation reaction of the latex surface was tested using various compounds of metal elements having three or more valences, and it was found to be possible to produce a latex article having non-adhesive surface.

Furthermore, we tested for surface treatment of the latex base substance with various organic crosslinking agents that crosslink the carboxy group of the carboxylated latex in the same manner as with the metal compound having three or more valences. As the result, it was confirmed that the organic crosslinking agent gave non-adhesive latex products as with the tri- or more valent metal compound. Emulsion type crosslinking agents tended to give high effect, and, even if considered as the crosslinking agents of the same type, water soluble organic crosslinking agents showed low effect. Moreover, the latex film formed by the dipping method by adding the water-soluble crosslinking agent to the coagulant raised problems that cracks developed, the film strength was poor and so forth. Presumably, the crosslinking agent diffused into the inside of the film to cause the so-called over-vulcanization, which brought forth the deterioration of strength.

We tested for addition of chemicals having coagulating effect to the emulsion latex in order to prevent the diffusion of the crosslinking agent, and confirmed that the undermentioned internal addition-use aluminum type inorganic crosslinking agents such as aluminates and aluminum hydroxide gels could be added to the emulsion latex without coagulating the carboxylated latex. In addition, the latex film that was not made non-adhesive in the treatment with a concentration as high as 1% of a water-soluble crosslinking agent such as oxazoline crosslinking agent, e.g. Epocross W (made by Nippon Shokubai), was made non-adhesive when such a compound having coagulation effect was added even in a concentration as extremely low as 10 ppm.

As to the system in which the internal addition-use aluminum type inorganic crosslinking agent such as aluminate or aluminum hydroxide gel was added to an emulsion latex, the effect of the diffusion of organic crosslinking agents was studied further. After forming a film with the coagulant containing organic crosslinking agent, the effect on the film strength of the temperature of the leaching stage was examined. As a result, the film strength lowered in the usual leaching stage temperature of 50 to 60°C, but the decrease of the film strength was not observed when the treatment of the leaching stage was conducted at 70 °C or more.

Presumably, unlike the tri- or more valent metal compound in the coagulation liquid, the crosslinking agent in the aqueous treatment solution, being poorly reactive with the carboxyl group in the latex at a low temperature, diffuses toward the Z-axis direction without staying on the surface of the latex product to cause the deterioration of the strength.

With the concentration of the organic crosslinking agent decreased, its effect on the adhesion and strength of the latex product was examined. Even with a concentration of the organic crosslinking agent as extremely low as 0.001% (2×10 ⁻⁴ part per latex solids), the latex product showed non-adhesion, and the strength of the product was equivalent with that of the untreated product. The effect of the organic crosslinking agents on the strength differs according to the properties thereof . Those which have a lower degree for the diffusion to the Z-axis direction, or those which react immediately on the surface of the latex and do not diffuse into the inside exert less influence on the product strength.

On the other hand, we conducted a wearing test for fingerstalls prepared in the test. The fingerstall obtained by vulcanizing the carboxylated latex with zinc oxide, although showing good results in the strength test, was found to be very poor in durability since the film developed cracks on wear from several hours to within one day. This is because the defect of ion crosslinking surfaced up.

We conducted a wearing test for the above-mentioned fingerstalls which were obtained by adding an internally added aluminum type inorganic crosslinking agent such as aluminate or aluminum hydroxide gel to an emulsion latex. Unexpectedly, they did not break when worn for a period from one day to one week. This is thought to be because, when the ion crosslinking by zinc oxide cleaves on use, the aluminum ion retaining crosslinking power repairs the cleaved points. Thus, by adding the internally added aluminum type inorganic crosslinking agent such as aluminate or aluminum hydroxide gel into the carboxylated latex, the non-adhesion treatment becomes possible even with an extremely low concentration of the organic crosslinking agent that hitherto has been incapable of non-adhesion treatment. Further, it becomes possible to produce latex products excellent in durability without sulfur vulcanization.

The retesting on tri- or more valent metal compound crosslinking agents gave similar results.

Other organic crosslinking agents for carboxyl group make the carboxylated latex product non-adhesive at a lower concentration, as in the said oxazoline type crosslinking agents.

Organic compounds which are not crosslinking agents for carboxyl group but are considered to react with carboxyl group have similar effects. Such compounds include glyoxal, polyamide compound, polyamide polyurea compound, polyamide polyureaglyoxal condensation reaction product, polyamine polyurea compound, polyamideamine polyurea compound, polyamideamine compound, polyamideamine epihalohydrine condensation reaction product, polyamideamine formaldehyde condensation reaction product, polyamine epihalohydrine condensation reaction product, polyamine formaldehyde condensation reaction product, polyamide polturea epihalohydrine condensation reaction product, polyamide polyurea formaldehyde condesation reaction product, polyamine polyurea epihalohydrine condensation reaction product, polyamine polyurea formaldehyde condensation reaction product, polyamideamine polyurea epihalohydrine condensation reaction product, and polyamideamine polyurea formaldehyde condensation reaction product. These compounds have been developed as waterproof endowing agent, sizing agent, printability improver, wet strength enhancing agent, and paper strength reinforcing agent of papers, and are common in that any of them is a chemical for controlling hydrogen bonds in the paper.

Next, we studied compounds such as monofunctional epoxy compounds and monofunctional amines that do not crosslink with the carboxyl group of the latex but react with the carboxyl group to inhibit the formation of hydrogen bond derived from the carboxyl group.

As a result, it was confirmed that these compounds, which are considered to bond to a carboxyl group to lead to hydrophobicity, afford similar effects to those of the before-mentioned compounds.

We studied on sizing agents used in the field of paper as chemical to block carboxyl groups. The typical example of the sizing agent is the rosin type, and the main ingredient of rosin is abietic acid. The rosin coats pulp fibers and exhibits excellent hydrophobicity at that time. The contact angle of rosin and water is as large as 53° , and if aluminum is bonded the contact angle becomes as extremely large as 130°. Thus, the rosin gives a large effect as hydrophobicity endowing agent. The rosin sizing agent was tested in the same manner as the above-mentioned, and the effect as a blocking agent of carboxyl group was confirmed. The effect of the sizing agent is thought because it coats the latex surface physico-chemically or physically into hydrophobicity, and it is estimated that aluminum ion plays a big role.

Further, in recent years, as the sizing agent for neutral paper there are used alkylketenedimer(AKD), alkenylsuccinic acid anhydride(ASA), cationic sizing agent, and the like. These neutral sizing agents also had a similar effect. Regarding the hydrophobicity effcet of AKD and ASA, it is said generally that they create hydrophobicity by chemically bonding to a hydrophilic group. But, there is a view that they cause autolysis on the surface of fiber to lose hydrophilicity and the whole compound becomes hydrophobic. In this time, the hydrophilic group works as anchor. In any event, it is evident that the relevant sizing agent blocks the carboxyl group on latex surface chemically, physico-chemically or physically to generate hydrophobicity.

We also studied on making the surface of latex products hydrophobic chemically or physically, taking the above-mentioned hydrophobicity effect into consideration. First, we had an eye to the hydrophobic group of surfactants and examined on nonionic surfactants. As the results, it was found that HLB, an index of hydrophilicity and hydrophobicity, could not interpret the degree of non-adhesion. Further, cationic and amphoteric surfactants do not have even such an index. We thought it convenient to determine the propriety by conducting adhesion test 1 or 2 as shown in the below-mentioned examples. The surfactant that showed non-adhesion effect is called hereinafter a non-adhesive surfactant.

The anionic surfactant has a similar effect as the said surfactant. However, the anionic surfactant loses the function of surfactant as soon as it forms a metal soap with polyvalent metal salts derived from a coagulant. Many of the metal salts of the anionic surfactants have adhesivenss, and the treatment with an anionic surfactant sometimes gives an adverse effect. To grasp the characteristics of the compound itself, it is necessary to conduct the adhesion test.

For the surfactant to exhibit the non-adhesion effect, it is necessary to add an internally added aluminum type inorganic crosslinking agent such as aluminate or hydroxyaluminum gel directly into an emulsion latex.

We further studied, based on the above-mentioned findings, the method of directly adding a carboxyl-group blocking agent into a carboxylated latex. When an article is made by the dipping method, the latex film after the dipping is extracted with hot water on the leaching stage. Accordingly, the carboxyl-group blocking agent incorporated in the latex liquates out on the leaching stage to fail in making the carboxylated latex product non-adhesive. On the other hand, when the carboxyl-group blocking agent is an emulsion type, when it reacts with a carboxylated emulsion latex, or when it is adsorbed on a carboxylated emulsion latex, it reacts with a bivalent metal salt, preferably calcium salt, in the coagulant and it dose not liquate out. In case where there is a special condition that the caboxyl group blocking agent does not liquate out, for example, a condition that the calcium soap formed above has non-adhesion effect or a condition that the carboxyl-group blocking agent does not liquate out by reacting with the aluminum type inorganic crosslinking agent such as aluminate or aluminumhydroxide gel added in the carboxylated emulsion latex, only incorporating the carboxyl-group blocking agent into the carboxylated emulsion latex gives a non-adhesive product.

As mentioned above, it is possible by using the present invention to fabricate the latex product which is non-adhesive in one or both surfaces thereof. The surfaces of such products do not attach together even when each surface comes in contact under heating during and after the manufacture of them. Accordingly, a product can be produced that has never existed until now.

One of such products is a non-adhesive fingerstall mechanically wound from its mouth before removing from the dipping former. The fingerstall wound from the mouth has been existed until now, and its usefulness has been recognized because of its easy to wear. However, the both surfaces of the fingerstall, a latex product, are intrinsically adhesive, and in order to manufacture the wound product, the fingerstall had previously to be made non-adhesive by a treatment such as powdering and post-chlorination, followed by winding with human hands. This made it impossible to maintain the cleanness of the product in a high level and it was difficult to use such a product in a workplace where precisely processed articles are manufactured. In contract, the present invention, which is able to make the both surfaces of a formed latex non-adhesive, can provide a method to mecahnically wind a fingerstall on a dipping former and can maintain the cleanness of the product to a high degree. Recently, a thin fingerstall is desired since wearing a thick one tends to cause tiredness. The thinner the fingerstall the more difficult to wear it, so desired is a wound fingerstall that is thin, powder-free, non-adhesive and clean. By utilizing the property that the both surfaces of the fingerstall are non-adhesive, a fingerstall with wound mouth can easily be manufactured. In manufacturing the fingerstall, the top of the stall is left adhesive without providing the layer of carboxyl-group blocking agent, the whole is wound up, and then unwound. By this, the adhesive portion remains as wound mouth. Hitherto, the wound mouth has been formed first by winding up only the top of the stall to make the wound mouth, and thereafter the stall must have been released from the former in another step. The wound mouth facilitates wear and removal of the stall and is highly desired for flat products. Further, after formation of the wound mouth by the conventional method, a non-adhesion treatment may be conducted. This procedure is also applicable to the wound fingerstall described in the preceding paragraph, thereby providing the fingerstall with wound mouth. The present invention relates to:
(1)A non-adhesive carboxylated latex product incorporated with a carboxyl-group blocking agent,
(2)A non-adhesive carboxylated latex product having a layer treated with a carboxyl-group blocking agent on one or both surfaces of a carboxylated latex product or a carboxylated latex product incorporated with a carboxyl group blocking agent,
(3)A non-adhesive carboxylated latex product according to (1) or (2), wherein the carboxylated latex is NBR, SBR, CR or MBR,
(4)A durable, non-adhesive carboxylated latex product according to (1) to (3), wherein the carboxylated latex is added and crosslinked with an internally added aluminum-type inorganic crosslinking agent, (5)A non-adhesive carboxylated latex product according to (1) to (4), wherein the latex product is a dip product,
(6)A non-adhesive carboxylated latex product according to (5), wherein the dip product is a fingerstall, gloves, balloon or condom,
(7)A non-adhesive carboxylated latex product according to (1) to (6), wherein the carboxyl-group blocking agent is a metal element crosslinking agent having three or more valences,
(8) A non-adhesive carboxylated latex product according to (7), wherein the metal element crosslinking agent having three or more valences includes at least one selected from aluminum, titanium or zirconium compounds,
(9)A non-adhesive carboxylated latex product according to (1) to (6), wherein the carboxyl-group blocking agent is an organic crosslinking agent for the carboxyl group of the carboxylated latex,
(10)A non-adhesive carboxylated latex product according to (9), wherein the organic crosslinking agent for the carboxyl group includes at least one selected from aziridine compounds, epoxy componds, blocked isocyanates, oxazoline compounds, carbodiimido compounds, melamineformaldehyde resins, ureaformaldehyde resins, isocyanates, phenolformaldehyde resins, glycols, polyols, diamines, polyamines, hexamethoxymethylmelamines and methylolacrylamides,
(11)A non-adhesive carboxylated latex product according to (1) to (6), wherein the carboxyl-group blocking agent includes at least one selected from glyoxals, polyamide compounds, polyamide polyurea compounds, polyamine polyurea compounds, polyamideamine polyurea compounds, polyamide polyurea glyoxal reaction products, polyamideamine compounds, polyamideamine epihalohydrine condensation reaction products, polyamideamine formaldehyde condensation reaction products, polyamine epihalohydrine condensation reaction products, polyamine formaldehyde condensation reaction products, polyamide polyurea epihalohydrine condensation reaction products, polyamide polyurea formaldehyde condensation reaction products, polyamine polyurea epihalohydrine condensation reaction products, polyamine polyurea formaldehyde condensation reaction products, polyamideamine polyurea epihalohydrine condensation reaction products,and polyamideamine polyurea formaldehyde condensation reaction products,
(12)A non-adhesive carboxylated latex product according to (1) to (6), wherein the carboxyl-group blocking agent includes at least one selected from monofunctional amines, monofunctional epoxy compounds, monofunctional isocyanates and monofunctional blocked isocyanates,
(13)A non-adhesive carboxylated latex product according to (1) to (6), wherein the carboxyl-group blocking agent is a sizing agent,
(14)A non-adhesive carboxylated latex product according to (1) to (6), wherein the carboxyl-group blocking agent is a non-adhesion surfactant,
(15)A method for producing a non-adhesive carboxylated latex product according to (1) to (14), wherein one or both surfaces of the latex product are brought into contact with one or more of the carboxyl-group blocking agent solutions described in (7) to (14) to attach the carboxyl-group blocking agent to the latex surface,
(16) A method for producing a non-adhesive carboxylated latex dip product, characterized in that there is used a solution of a mono- or bi-valent external coagulant for carboxylated latex which is mixed with or dissolved in one or more of the carboxyl group blocking agents described in (7) to (14),
(17) A method for producing a non-adhesive carboxylated latex dip product, characterized in that a dipping former is dipped and deposited with one or more of the carboxyl-group blocking agent described in (7) to (14), dipped and deposited with a mono- or bi-valent external coagulant, and then dipped in a latex,
(18) A method for producing a non-adhesive carboxylated latex dip product, characterized in that a dipping former is dipped and deposited with one or more of the carboxyl-group blocking agents described (7) to (14), then dipped in a latex liquid to form a latex film, further dipped in a mono- or bi-valent external coagulant solution, and subsequently dipped in the carboxylated latex again,
(19) A method for producing a non-adhesive carboxylated latex dip product, characterized in that a dipping former is dipped in a mixture of one or more of the carboxyl-group blocking agents described in (7) to (14) and a carboxylated latex stable to the blocking agent to form a latex film, further dipped in a mono- or bi-valent external coagulant solution, and thereafter dipped in the carboxylated latex again,
(20) A method for producing a non-adhesive carboxylated latex dip product, characterized in that a dipping former is dipped in a mono- or bi-valent coagulant suspension for carboxylated latex which contains, as the carrier, fine powder of one or more of the carboxyl group blocking agents described in (7) to (14) that is hardly soluble or insoluble in water or alcohol, and subsequently dipped in the carboxylated latex liquid.
(21)A non-adhesive fingerstall, wherein the fingerstall described in (6) has a shape mechanically wound from the mouth before removed from the dipping former,
(22)A non-adhesive fingerstall according to (6) or (21) which has a wound mouth,
(23)A method for producing a non-adhesive fingerstall with wound mouth according to (22), characterized in that an adhesive portion is provided on the upper part at the time of dipping and then winding is conducted, (24) A method for producing a non-adhesive fingerstall according to (6) or (21), characterized in that the outside surface is treated with a carboxyl group blocking agent after a wound mouth is provided.

The term carboxyl group blocking agent as used herein means a substance which blocks a carboxyl group chemically, physico-chemically or physically and inhibits the formation of a hydrogen bond derived from the carboxyl group. Specifically, it means a compound which can make the carboxylated latex non-adhesive in the adhesion test 1 or 2 of the examples. Further, the non-adhesion surfactant refers to a surfactant which can make the carboxylated latex non-adhesive in the adhesion test 1 or 2.

### [Brief Description of the Drawings]

Fig.1 is a perspective view of the dipping former transferring apparatus used in the examples. 1, chain; 2, guide rail; 3, dipping former; 4, rod; and 5, guide.
Fig.2 shows the trial apparatus for manufacturing the fingerstall used in the examples. 6, dipping tank; 7, drying furnace; 8, winding machine; and 9, unwinding machine.
Fig.3 shows the main parts of the winding machine. 10, rolling brush; and 11, film.
Fig.4 shows the main parts of the unwinding machine.

### [Best Mode for Carrying out the Invention]

The present invention is described in more detail hereinafter. The first non-adhesive carboxylated latex product of the invention is a latex product in which non-adhesiveness properties have been afforded by directly adding a carboxyl group blocking agent into an emulsion latex.

The second is a carboxylated latex product having a treatment layer of the carboxyl group blocking agent on one or both surfaces of the carboxylated latex base. Both products are intrisically non-adhesive. If special use characteristics such as dry or wet wearing properties are required, an outer layer can be provided further on the outside surface according to known technology. The latex products which need prevention against adhesion include, but are not limited to, dip products such as balloons, gloves, fingerstalls and condoms, extruded products such as rubber threads and rubber pipes, cast-molded products such as balloons and toys, and whole rubber products or products having rubber surface such as rubber sheets, hoses and draw clothes.

The kind of the carboxylated latex used herein is not limited, but among elastomer latexes of NBR, SBR, CR, MBR and the like, ones subjected to carboxylation are preferable.

There is no limitation about a so-called vulcanizing agent added to the carboxylated latex, and normal vulcanizing agents can be used: e.g., agents for sulfur, peroxide, zinc oxide or radiation vulcanization. However, when the latex product is used in the field where the reaction of a metal and sulfur is undesirable, for example in the field of precision instrument, sulfur cannot be used as a vulcanizing agent. For such use the product vulcanized by zinc oxide is generally used, but there is a risk that it may break during wear because of ion crosslinking. Especially for thin gloves or fingerstalls durability is required. By adding an internally added aluminum type inorganic crosslinking agent into the carboxylated latex, durability is greatly improved. At the same time, the range of compounds that can function as carboxyl group blocking agent becomes sharply wide and the amount to be added can sharply be decreased. Here, the internally added aluminum type inorganic crosslinking agent refers to an inorganic aluminum compound which do not coagulate the latex at ordinary temperature even when directly added to the carboxylated latex and which crosslinks the carboxylated latex when heated. The typical examples thereof are alkali metal aluminates such as water-soluble sodium aluminate, alkaline earth metal aluminates such as sparingly soluble calcium aluminate, and aluminum hydroxide gel. Also included are the groups of various aluminum compounds such as magnesium aluminate metasilicate, synthetic hydrotalcite, aluminosilicagel and aluminosilicate. In other words, they are compounds which do not dissociate into aluminum ion when added but combine with a carboxyl group of the latex through ionic crosslink when heated. The aluminum-ion crosslink of these compounds is considered to occur via aluminum hydroxide.

As the latex product is a dip product, it is preferable to use an external coagulant, and any of the usually used external coagulant can be employed. Usually, mono- or bi-valent metal salts are used, and the monovalent salts include ammonium salts. Here, the external coagulant means one that is not directly incorporated into the latex but used in the coagulation- dipping process including depositon to the dipping former.

The carboxyl group blocking agent used in the invention is a compound which acts on a carboxyl group of the carboxylated latex chemically, physico-chemically or physically, thereby resulting in hydrophobicity which inhibits the formation of a hydrogen bond derived from the carboxyl group so as to make the latex product non-adhesive.

The first carboxyl group blocking agent is a tri- or more valent metal compound. Although any agent can be used if it has three or more valences, it is necessary to fully consider safety and side effect (discoloration) of the compound.

The example of the tri- or more valent metal compound usable as the external crosslinking agent in the invention includes one which is soluble in water or alcohol and one which is sparingly soluble or insoluble. The soluble trivalent metal compound includes aluminum salts, ferric salts, chromate and thorium salts. Practically, suitable are aluminum salts such as aluminum chloride, aluminum nitrate, aluminum sulfate and aluminum acetate.

Polyaluminumchloride(PAC) and water-soluble polyaluminum hydroxide are more preferable because they have three or more valences. Especially, water-soluble polyaluminumhydroxide gives the effect at its low concentration comparable to that of the organic crosslinking agent, as shown in the examples. If the metal is an amphoteric element, its metal acid salt is usable and sodium aluminate is a typical example. Observation suggests that sodium aluminate is converted into aluminum hydroxide on the film latex surface and then causes crosslinking.

The metal compound sparingly soluble or insoluble in water or alcohol that acts as the external crosslinking agent includes oxides, hydroxides and alkaline earth metal salts. The typical examples are aluminum hydroxide, calcium aluminate and magnesium aluminate. The aluminum compound includes various substances such as aluminosilicates, which fall into the invention. The usual crystalline aluminum hydroxide is hardly involved in the crosslinking reaction. The so-called amorphous aluminum hydroxide, when dispersed with a ball mill and made to have a large specific surface, is involved in the crosslinking reaction, and is absorbed on the latex film surface formed. Further, calcium aluminate and magnesium aluminate are absorbed on the latex surface in the same way.

As used herein, the external crosslinking agent refers to the one that is not directly incorporated into the based emulsion latex and crosslinks the latex by contacting with the surface of the emulsion latex or latex film.

The tetravalent metal compound used in the invention includes zirconium compounds such as zirconyl nitrate, ammonium, zirconyl carbonate zirconyl carbonate W, ammonium carbonate zirconuim oxychloride, and titanium compounds such as titanium lactate, titanium maleaic anhydride and titanium oxalate.

In the case of a tri- or more valent water soluble metal salt, the dissociated metal ion is cationic and reacts with the anion of carboxyl group even at low temperature. Further, according to the Shultz-Hardy Law, it has a strong crosslinking force. Because of this, the difussion of the metal ion having three or more valences is very weak, and the reduction of strength of the latex film is minimumized. Accordingly, the quality of the final product is kept favorable even if the concentration of the metal salt is considerably high.

The tri- or more valent metal compounds used in the invention include organic compounds. The typical examples are carboxylic acid salts, respectively the above-mentioned aluminum acetate, zirconium acetate, titanium lactate, titanium maleic anhydride, titanium oxalate and titanium lactate. They are not limited to carboxylic acid salts.

The concentration of the tri- or more valent metal external crosslinking agent varies depending on the kind of latex, the amount of functional groups involved in the reaction, the kind and quantity of an emulsifier or dispersant, the kind of a crosslinking agent, a treatment method, and the crosslinking agent supporting ability of a dipping former. Preferably, it is in the range of 0.1 to 5% (as metal oxide corresponding to the metal element). When the agent is used by incorporation into the external coagulant, it exhibits a sufficient effect in the range of 0.01 to 0.5%. In the case of working it on the formed latex film and in the case of providing the external coagulant layer on the external crosslinking agent layer, a preferable concentration is 0.2 to 1%. When the agent is used through incorporation into the external coagulant, it replaces monovalent cations of the latex, usually ammonium ion, potassium ion and sodium ion, to form a crosslink, whereas one other than the above is used as a coagulant and necessary to be replaced with a bivalent cation, usually calcium ion, bonded to the latex to form a crosslink.

In the system in which the internally added aluminum type inorganic crosslinkingn agent is directly added to the latex, the concentration of the external crosslinking agent can be decreased.

The second carboxyl group blocking agent is an organic crosslinking agent for the carboxyl group of the carboxylated emulsion latex. Any kind is usable as long as it is an organic crosslinking agent capable of crosslinking a carboxyl group. The typical organic crosslinking agent usually used includes, but are not limited to, aziridine compound, epoxy compound, blocked isocyanate, oxazoline compound, carboimide compound, melamineformaldehyde resin, ureaformaldehyde resin, isocyanate, phenolformaldehyde resin, glycol, polyol, diamine, polyamine, hexamethoxymethylmelamine and methlolacrylamide. (see Newest Applied Technology of Latex Emulsion, page 323, Motoharu Okikura (ed) published by Chunichisha, Japan)

The organic crosslinking agent for carboxyl group reacts at a considerably high temperature. However, according to the invention, since its amount is very small, it shows the effect at a temperature of about 90 to 120°C. However, if time lapses on the stage of a low temperature, the crosslinking agent diffuses in the Z-axis direction before strength of the latex film is expressed, and competes with the so-called latex vulcanizer such as zinc oxide to restrain vulcanization, resulting in lowering the film strength. Particularly, the temperature of the leaching stage of a dipping method is preferably 65 °C or more, more preferably 70 to 85 °C. The temperature of 85 °C or more is not preferable because bubbles are generated between the dipping former and the film. When the concentration of the organic crosslinking agent in the coagulant is high, diffusion of the crosslinking agent becomes large and the strength is lowered. But, an emulsion type crosslinking agent or highly reactive crosslinking agent diffuses weakly, and the degree of the diffusion also differs depending on the solubility of the crosslinking agent in the coagulant solution. Therefore, it is preferable to determine optimal conditions through an adhesion test. An almost suitable concentration can be determined by carrying out the test at two levels of effective ingredient concentration, 0.025% and 0.0025%. The amount of a crosslinking agent to make the carboxylated emulsion latex product non-adhesive is extremely small.

Organic compounds which do not belong to the crosslinking agent for carboxyl group but are thought to react with a carboxyl group, also have the similar effect. As such effective compounds are the following: glyoxal, polyamide compound, polyamidepolyurea compound, polyamidepolyureaglyoxal condensation reaction product, polyaminepolyurea compound, polyamideaminepolyurea compound, polyamideamine compound, polyamideamineepihalohydrine condensation reaction product, polyamideamineformaldehyde condensation product, polyamineepihalohydrine condensation reaction product, polyamineformaldehyde condensation reaction product, polyamidepolyureaepihalohydrine condensation reaction product, polyamidepolyureaformaldehyde condensation reaction product, polyaminepolyureaepihalohydrine condensation reaction product, polyaminepolyureaformaldehyde condensation reaction product, polyamideaminepolyureaepihalohydrine condensation reaction product, and polyamideaminepolyureaformaldehyde condensation reaction product. Many of these compounds were developed as waterproof endowing agent, printability improver, wet strength improver, paper strength reinforcing agent of papers, and they are common in that they were developed as chemicals to inhibit the formation of hydrogen bonds in paper. The reaction conditions and the concentration of the compounds are similar to those of the organic crosslinking agent, providing comparable effects.

The methods of producing the above-mentioned compounds are not limited, but the general methods are described hereinafter.

The polyamide compound (also called polyamideamine compound) is obtained by dehydration condensation reaction of a polyamine and a compound having carboxyl group. The polyamidepolyurea, polyaminepolyurea, polyamideaminepolyurea and polyamideamine compounds are reaction products between polyalkylenepolyamine or alkylenepolyamine, urea, and dibasic carboxylic acid. The compounds can be modified with a small amount of aldehyde, epihalohydrine or α,γ-dihalo-β-hydrine. The method of their production is described in Japanese Patent Publication (Kokoku) No.59-32597 and Japanese Patent Application Laid-Open (Kokai) No.4-10097.

The polyamideamine-epihalohydrine condensation reaction product, polyamideamine-formaldehyde condensation reaction product, polyamine-epihalohydrine condensation reaction product, polyamineformaldehyde condensation reaction product, polyamidepolyureaepihalohydrine condensation reaction product, polyamidepolyureaformaldehyde condensation reaction product, polyaminepolyureaepihalohydrine condensation reaction product, polyaminepolyureaformaldehyde condensation reaction product, polyamideaminepolyureaepihalohydrine condensation reaction product and polyamideaminepolyurea-formaldehyde condensation reaction product, are reaction products between polyalkylenepolyamine, urea, dibasic carboxylic acid, and epihalohydrine or formaldehyde. The manufacture methods are described in Japanese Patent Publication (Kokoku) No.52-22982, Japanese Patent Publication (Kokoku) No.60-31948, Japanese Patent Publication (Kokoku) No.61-39435 and Japanese Patent Application Laid-Open (Kokai) No.55-127423.

The sizing agent for paper is a chemical which makes the hydrophilic group of paper hydrophobic and prevents the soak of ink, and also such chemical makes the carboxylated latex product non-adhesive. It is considered that the chemical makes carboxyl group hydrophobic chemically, physico-chemically or physically. There is no established theory for the hydrophobicity-forming mechanism, but the sizing agent gives a large and stable effect since it has been developed as a sizing agent of paper.

The sizing agent for paper includes internally added sizing agents and surface sizing agents. The invention can use any sizing agent on the like as long as it exhibit the function.

The internally added sizing agent is classified into an acid sizing agent, a neutral sizing agent and an acid/neutral sizing agent (Japanese Patent Application Laid-Open (Kokai) No.11-61682).

The acid sizing agent includes rosin sizing agent, fatty acid soap sizing agent, synthetic sizing agent and petroleum resin sizing agent.

The rosin sizing agent includes rosins and rosin derivatives. The rosins refer to gum rosins, wood rosins and tol oil rosins which contain, as the main component, resin acids such as abietinic acid, palustric acid, neoabietinic acid, pimaric acid, isopimaric acid and dehydroabietinic acid.

The rosin derivatives include hydrogenated rosins, polymerized rosins, modified rosins, fortified rosins,rosin esters and fortified rosin esters.

The modified rosins include (alkyl)phenol-formalin resin modified rosins, xylene resin modified rosins, aldehyde modified rosins and styrene modified rosins.

The fortified rosins are obtained by heating and reacting the said rosins and α,β-unsaturated carboxylic acids.

The rosin esters are produced using rosins and polyhydric alcohols by the known esterification.

The fortified rosins are obtained by successively or simultaneously reacting the said rosins and/or the said modified rosins with known polyhydric alcohols and α,β-unsaturated carboxylic acids.

The fatty acid soap sizing agent is a sizing agent obtained by neutralizing a fatty acid having the carbon number of about 8 to 24 such as palmitic acid or stearic acid and a mixture thereof with an alkali.

The synthetic sizing agent includes the sizing agent obtained by neutralizing with an alkali a substituted succinic anhydride obtained by reacting an oligomer, dimer or tetramer, of isobutene and a mixture thereof with maleic anhydride.

The petroleum resin sizing agent includes one obtained by modifying a petroleum resin with an unsaturated carboxylic acid such as maleic acid. The petroleum resin includes C5 petroleum resin obtained by polymerizing C5 olefin such as 1,3-pentadiene or isoprene, C9 type petroleum resin obtained by polymerizing C9 olefin such as coumarone or indene, C5/C9 copolymer type petroleum resin obtained by copolymerizing C5 olefin and C9 olefin, and dicyclopentadiene type petroleum resin obtained by polymerizing dicyclopentadiene or its derivative.

The neutral sizing agent includes alkylketenedimer type, alkenylketenedimer type, alkenyl succinic anhydride type and neutral rosin type sizing agents.

The alkylketenedimer type or alkenylketenedimer type sizing agent can be usually produced by emulsifying an alkylketene dimer or alkenylketene dimer which is prepared by treating a saturated or unsaturated fatty acid chloride having the carbon number of about 12 to 24 with a base such as triethylamine to make a dimer.

The alkenyl succinic anhydride type sizing agent can be prepared by emulsifying an alkenyl succinic anhydride obtained by addition reaction of a terminal and /or internal olefin having the carbon number of about 12 to 24 with maleic anhydride.

The neutral rosin sizing agent includes polyhydric alcohol esters of rosins, and emulsions made by dispersing petroleum resin-containing substances in water.

The polyhydric alcohol esters of said rosins include reaction products containing rosin esters obtained by reacting rosins, with (a)at least one chemical belonging to the scope of polyhydric alcohols and (b)at least one chemical belonging to the scope of α,β-unsaturated carboxylic acid or its derivative.

As the acid/neutral sizing agent there are known cationized fatty acid bisamide type, cationized petroleum resin type, cationized polymer type and α-hydroxycarboxylic acid type sizing agent.

The cationized fatty acid bisamide type and cationized petroleum resin type sizing agent are synthesized by reacting a maleic acid adduct of a fatty acid having about 12 to 24 carbons or a petroleum resin with a polyamine such as diethylenetriamine and triethylenetetramine and a mixture thereof, followed by reaction with epichlorohydrine.

The cationic polymer type sizing agent is usually synthesized by radical polymerizing a cationic vinyl monomer such as dimethylaminoethylmethacrylate and a hydrophobic monomer such as styrene, acrylonitrile or alkyl(meth)acrylate in water and /or in an organic solvent.

The α-hydroxycarboxylic acid type sizing agent is made by reacting a higher alcohol or higher amine with an oxyacid such as citric acid. The surface sizing agent generally comprises a hydrophobic portion and an anionic portion such as carboxyl group. The surface sizing agent is obtained by copolymerizing a hydrophobic monomer and an anionic monomer such as α, β -unsaturated monocarboxylic acid, α, β -unsaturated dicarboxylic acid and unsaturated sulfonic acid. (see Japanese Patent Application Laying-Open (kokai) No.12-45197)

The example of the surface sizing agent which consists of a copolymer of a hydrophobic monomer and an anionic monomer includes styrene-(meth)acrylic acid copolymer, styrene- (meth)acrylic acid-(meth)acrylic acid ester copolymer, styrene-maleic acid copolymer, styrene-maleic acid-maleic acid halfester copolymer, (di)isobutylene-maleic acid copolymer, (di)isobutylene-maleic acid-maleic acid halfester copolymer and salts of these.

The surface sizing agent other than the above-mentioned includes alkylketene dimer, alkenyl succinic acid(anhydride), styrene-acrylic acid copolymer, acrylate-acrylonitrile copolymer, styrene-dialkylaminoalkyl(meth)acrylate copolymer, and reaction product thereof with epihalohydrine.

The carboxyl group blocking agent is not necessarily a crosslinking agent for carboxyl group. If it is a substance which acts on carboxyl group chemically or physico-chemically further or physically, which makes the site hydrophobic and hinders the formation of a hydrogen bond, the agent makes the carboxylated latex product non-adhesive.

Concretely, even the compounds that cannot form crosslinking structure with carboxyl group, for example, monofunctional amines, monofunctional epoxy compounds, monofunctional isocyanates and monofunctional blocked isocyanates, make the carboxylated emulsion latex product non-adhesive. Since they have one functional group, crosslinking does not occur. However, if these compounds have hydrophobic group they react with a carboxyl group to result in hydrophobicity.

As to the amines, any of primary, secondary, tertiary and quarternary amines gives the effect.

The surfactant is composed of hydrophilic group and hydrophobic group.

If the hydrophilic group is arranged to the surface of the carboxylated emulation latex product and the hydrophobic group to the outside, supposedly the carboxyl group of the product surface is blocked to form hydrophobicity and to make the surface non-adhesive. Accordingly, it is supposed that the degree of hydrophobicity of the surfactant controls the degree of non-adhesion of the product. Whether the surfactant takes up the position on the product surface with the hydrophobic group directed to the outside is determined by the physical and chemical properties of the surfactant itself, the properties of the latex, addition or no addition of aluminum compounds and the like. And it is difficult to find a general rule. So, it is necessary to select non-adhesion surfactant through the adhesion test 2 shown in the examples. The results of the test were that the surfactant showed the non-adhesion effect in the case that the internal addition-use aluminum type inorganic crosslinking agent such as aluminum hydroxide gel and aluminate was added into the emulsion latex. The presumable reason for this is that the surfactant, viewed in the light of its properties, diffuses the latex film. The internal addition-use aluminum type inorganic crosslinking agent is thought to suppress diffusion of the surfactant.

Regarding to the nonionic surfactant, a general tendency cannot be found. However, it is seen that the nonionic surfactant having a high HLB shows a lowered non-adhesion effect and that the nonionic surfactant of amine and amide types shows a high non-adhesion effect.

In the cationic surfactant and the amphoteric surfactant, the cation of the surfactant and the anion of the carboxyl group combine with ion linkage. Hence, many of these give good results when subjected to the above-mentioned adhesion test. However, since the both surfactants make chemical reaction with carboxyl group at low temperatures, they influence the formation of the latex film in the dipping step, if they are added in the coagulant for use. That is, according to the kind and concentration of the surfactant, fine wrinkles are generated toward the direction of the dipping, and sometimes cracks are generated. Further, if the surfactant diffuses into the inside, it competes with the so-called latex vulcanizing agent such as zinc oxide and hinders vulcanization to cause decrease of the strength. Consequently, countermeasures are needed of restraining the surfactant concentration low to suppress diffusion, accelerating vulcanization by raising the temperature of the drying and leaching process and so forth, and these are big drawbacks. From the above-mentioned point the adhesion test is needed.

It is unclear how the anionic surfactant gives the non-adhesion effect. Generally, the anionic surfactant is used as an emulsifier in synthesizing the carboxylated emulsion latex. Moreover, dodecybenzenesulfonic acid, for example, reacts with metal salts in the coagulant at the dipping, most of it being converted to metal soap, and it loses the function as surfactant. Metal soaps, as seen from the fact that they are used as lubricating oil, have more or less adhesion. But, among the anionic surfactants some show excellent non-adhesion effect. There are many effective surfactants among those that have amine and amide group in the structure and are considered to react with carboxyl group and those that have a polycyclic structure and are considered to be highly hydrophobic. For example, the use of naphthalenesulfonic acid formaline condensation reaction products or alkylnaphthalenesulfonic acid formaline condensation reaction products is effective. The above-mentioned rosin sizing agent is interpreted to belong to this category.

The surfactant, because of its properties, has a great effect on each manufacturing step of the carboxylated emulsion latex product and on the properties of the product. Therefore, in order to judge whether a surfactant is usable or not, it is necessary to confirm the non-adhesion effect by conducting the adhesion test and at the same time to study the effect on film forming and quality.

There are a variety of methods for manufacturing the non-adhesion latex product of the invention.

In the present case of a dip product, by depositing one or more carboxyl group blocking agents and a usual external coagulant onto the dipping former and then bringing into contact with the emulsion latex, coagulation of the latex and treatment with the blocking agent can be conducted at the same time.

There are four methods for depositing the carboxyl-group blocking agent on the dipping former. The first method is one wherein a usual external coagulant consisting of mono- or bi-valent metal salt and one or more of the carboxyl group blocking agent of the invention are mixed and dissolved and deposited onto the dipping former. The second method is one wherein the carboxyl-group blocking agent of the invention is deposited onto the dipping former and thereafter a usual external coagulant is deposited onto the formed layer. Modifying this method gives a method wherein the carboxyl group blocking agent is incorporated into the emulsion carboxylated latex, the dipping former is dipped in said formulation liquid to form a thin latex film, the former is dipped in mono- or bivalent external coagulation solution, and then the former is dipped in the carboxylated latex solution again.

The third method is one wherein one or more of the powdery carboxyl blocking agents of the invention is used as support, said carboxyl group blocking agent being suspended in a usual external coagulant solution, and then deposited onto the dipping former. This method does not give a perfect non-powder product, but, since the carboxyl-group blocking agent reacts with the latex and is absorbed, the powder can be reduced to such a degree as unable to be perceived on the finished product.

By immersing such dipping former in the emulsion latex, there is obtained a dip product in which the inside surface contacting the former is non-adhesive.

The fourth method is one wherein a thin, carboxyl group blocking agent treated film of the latex is formed by using the carboxyl group blocking agent as an external coagulant, the external coagulant consisting of a mono- or bi-valent metal compound is deposited onto the film, and the former is dipped in the latex liquid again. With this method it is possible to make the inside surface non-adhesive, but it is feared that peeling may occur between layers of the product.

Further, the outside surface can be made non-adhesive by the above-mentioned methods.

Accordingly, by utilizing such a property, a product can be produced that has never existed until now.

One of such products is a non-adhesive fingerstall mechanically wound from its mouth before removing from the dipping former. The fingerstall wound from the mouth has been existed until now, and its usefulness has been recognized because of its easy to wear. However, the both surfaces of the fingerstall, a latex product, are intrinsically adhesive, and in order to manufacture the wound product, the fingerstall is previously made non-adhesive by a treatment such as powdering and post-chlorination, followed by winding with human hands. This makes it impossible to maintain the high cleanness of the product and it is difficult to use the conventional fingerstall in a workplace where precisely processed articles are manufactured. The present invention, however, which is able to make the both surfaces of the latex molding non-adhesive, can afford to mechanically wind the fingerstall on the dipping former and can maintain the high cleanness of the product.

Recently, a thin fingerstall is desired since wearing a thick one tends to cause tiredness. The thinner the fingerstall the more difficult to wear it, so desired is a wound fingerstall that is thin, powder-free, non-adhesive and clean.

By utilizing the property that the both surfaces of the fingerstall are non-adhesive, fingerstalls with wound mouth can easily be manufactured. In manufacturing the fingerstall, the top of the stall is left adhesive without providing the layer of the carboxyl group blocking agent, the whole is wound up, and then unwound. The portion having adhesion remains as a wound mouth. Hitherto, the wound mouth has been formed first by winding up only the top of the stall to make the wound mouth, and thereafter the stall has been released from the former in another step. The wound mouth facilitates wear and removal of the stall and is highly desired for flat products. Further, after formation of the wound mouth according to the prior art, non-adhesion treatment can be conducted to achieve the objective.

Further, the wound mouth can be provided for the wound fingerstall mentioned in the previous paragraph.

### [Example]

The present invention is explained in detail using examples hereinafter, but the invention is not limited to the examples. The percentage and the part shown in the examples are a weight percentage and a weight part unless otherwise indicated. Further, in the examples mentioned below, unless otherwise shown, the latex film was prepared by the dipping method from the viewpoint of easiness of the experiments and easiness of confirming the effect.

Examples 1-14, 86-88 and 90 are not within the scope of the present claims.

### (Preparation of Latex Film)

Using a test tube-like, glass-made dipping former for making fingerstall, a latex film was prepared. When an external coagulant and a carboxyl-group blocking agent of solution types were used, a sand-blasted glass-made dipping former was used. When a powdery carboxyl-blocking agent was used, a transparent glass-made dipping former not subjected to sand blasting was used. First, the dipping former was dipped in the external coagulation liquid to attach the liquid and dried. Next, the dipping former was dipped in a latex emulsion to form a latex film, and then drying, leaching and vulcanization treatment were conducted according to the conventional method.

In the case of treating the outside surface of the film, the dipping former affixed with the film is again dipped in a solution or suspension liquid of the carboxyl group blocking agent, followed by drying and leaching treatment.

As the latex, a carboxylated NBR, NIPOL LX-551, made by Nippon Zeon was used unless specially mentioned. The latex is not limited to this. The properties of LX551 are shown below.

| | |
|---|---|
| Solids | 45% |
| PH | 8.5 |
| Viscosity | 85 mPs |
| Gel content | 0% |
| Tg | - 14 °C |
| Combined AN content | 37% |

To 100 parts of the above-mentioned latex, fine zinc flower was added as a vulcanizing agent, and the mixture was subjected to the test.

### (Adhesion Test of Latex Film-1)

The adhesion of the latex film is highest when the film is heated in the water-containing state.

After the leaching step following the non-adhesion treatment of the outside surface in the film manufacturing process, the latex film is wound on the dipping former and removed from the dipping former as it stands. This sample is heated in a hot air dryer at 70 °C for 30 minutes, taken out and unwound after cooling. The film is thick because it is wound, and the sample is always in the wet condition during the test. Accordingly, if the both surfaces of the film are non-adhesive, unwinding is easy, and if adhesive, unwinding is difficult.

### Comparative Example 1

### (Preparation of External Coagulation Solution)

A solution of calcium nitrate tetrahydrate 150g and water or methanol 1000g was prepared to make an external coagulant(hereinafter called coagulant 1).

### (Manufacture of Latex Film)

A sand-blasted glass pipe is dipped in the aqueous external coagulant 1 and dried. The glass pipe attached with the external coagulant is dipped in a latex emulsion to form a latex film. The film affixed on the dipping former is dried softly at 50 °C for 3 minutes and further subjected to the leaching treatment for 3 minutes in the hot water of 70°C . Subsequently, the film is wound on the former and released from the former. The film is unwound and subjected to vulcanization treatment in a hot air dryer at 120 °C for 30 minutes to make samples for testing strength. For the adhesion test, the sample wound and removed from the former was presented as it stood. The test results of film strength and adhesion are shown in Table 1.

The sample after the adhesion test could not be unwound.

### (Example 1)

The film formed in Comparative Example 1 is leached, released from the former, unwound, dipped in an aqueous solution of polyaluminumchloride(which is an external crosslinking agent)containing 2.5% as alumina and immediately taken out. At this time the film surface already has lost adhesiveness and gives a feeling of smoothness. This film is dried at 70 °C for 3 minutes, further subjected to leaching treatment in the hot water at 70 °C for 3 minutes, and subjected to vulcanization treatment in the same manner as Comparative Example 1. The results of strength test of the film are shown in Table 1. For the adhesion test, the film, which underwent leaching treatment after non-adhesion treatment of the outside surface, was again placed on the former. The film was wound to make test samples. The sample after the adhesion test could easily be unwound.

### (Example 2)

The dipping former was dipped in a solution of polyaluminum chloride(which is an external coagulant)containing 2.5% as alumina and used in Example 1 , attached, then dried, next dipped in the external coagulant methanol solution 1, attached and dried. The dipping former was dipped in the latex liquid to form a film, then dried at 50 °C for 3 minutes and further subjected to leaching treatment at 50 °C for 3 minutes. Thereafter the former was again dipped in the aqueous solution of polyaluminum chloride used in Example 1 in order to prevent the adhesion of the film outside surface and subjected to leaching treatment and vulcanization treatment. The test results on the film strength are shown in Table 1. The sample after the adhesion test was able to be unwound easily. This shows that the inside surface contacting the dipping former has become non-adhesive. The outside surface is non-adhesive, as in Example 1.

### (Example 3)

Except that aluminum acetate containing 2.4% as alumina was used in place of polyalunimun chloride which is attached to the dipping former and in which the formed film is dipped, the latex film was prepared as with Example 2. Table 1 shows the test results on the strength of the film. The film wound in the adhesion test could easily be unwound.

### (Example 4)

Using aluminum nitrate containing 0.2% alumina and a solution containing calcium nitrate tetrahydrate 150g and methanol 1000g as external coagulation liquid and external crosslinking liquid, a film was produced. Then, there was conducted adhesion preventing treatment of the outside surface of the film according to the same operation as in Example 2. The film thickness was approximately equal to that of Comparative Example 1 and Example 2. The test results on the strength of the film are shown in Table 1. In this case too, as with Example 2, the sample after the adhesion test could easily be unwound.

### (Comparative Example 2)

The dipping former was dipped in the external crosslinking agent, polyaluminum chloride solution, used in Example 1, and a film was formed in the same manner as Comparative Example 1. The film thickness was only 0.03 mm, and polyaluminum chloride was found to be unsuitable as external coagulant. Using various aluminum compounds such as aluminum chloride, aluminum nitrate and aluminum acetate, the same tests as above were conducted. The results were the same.

### (Comparative Example 3)

The order of attaching the external crosslinking agent and the external coagulant in Example 2 was inverted, and dipping treatment was conducted so that the layer of polyaluminum chloride might come outside. The thickness of the film was only 0.04 mm, being similar to Comparative Example 2. This formulation was unsuitable for external coagulant formulation.

### (Reference Example 1)

Except that fine zinc flower was not added in the emulsion latex, a latex film was prepared in the same way as Example 3. As with Example 3, the film formed had a usual thickness, and the both surfaces of the film were non-adhesive. On the other hand, the strength of the film was extremely low. This fact can be interpreted as that the aluminum crosslinking layers are extremely thin and it does not contribute to the film strength.

### (Comparative Example 4)

Except that an aqueous solution of zinc nitrate containing 5% as zinc oxide was used in place of polyaluminum chloride, a latex film was prepared in the same way as Example 1. The film was adhesive, and it was impossible to carry out unwinding after the adhesion test. Zinc is bivalent and insufficient to make the film non-adhesive.

Table 1 shows the test results on the film strength.

### (Example 5)

An aqueous solution of sodium aluminate and an aqueous solution of calcium nitrate were mixed to prepare calcium aluminate. A solution of water/methanol (1/1) was prepared to make calcium aluminate 20g/1000g (calculated as anhydride) and calcium nitrate tetrahydrate 150g/1000g.

A transparent glass-made dipping former was dipped in said external coagulation liquid, a coagulant layer was formed on the former using caicium aluminate as support, and thereafter a latex film was prepared in the same way as Example 2.

On the dipping former calcium aluminate remains thinly, but when the inside surface after vulcanization is observed, the calcium aluminate is hardly recognized. The sample after the adhesion test could be unwound. The test results of the film strength are shown in Table 1.

When the dipping former is vigorously shaken immediately after the film is formed through dipping in the emulsion latex, the film breaks down. Under the layer broken down, a very thin aluminum treatment layer is recognized. Taking into consideration this fact and Reference Example 1, it is understandable that the aluminum treatment layer is a very thin layer.

### (Example 6)

Dry aluminum hydroxide gel made by Tomita Seiyaku Co. was dispersed with a ball mill for 24 hours and then added with water and methanol so that a suspension of aluminum hydroxide 20g (calculated as anhydride) in 1000g water/methanol(1/1) and calcium nitrate tetrahydrate 150g in 1000g water/methanol(1/1) is prepared. Thereafter a latex film was formed in the same way as Example 5. The test results on strength are shown in Table 1. The wound film could easily be unwound as with Example 4. In the process of heating the film it is observed that aluminum hydroxide is absorbed in the latex film.

### (Comparative Example 5)

There was prepared a suspension solution of water/methanol(1/1) having light calcium carbonate 100g/1000g, bentonite 50g/1000g and calcium nitrate tetrahydrate 150g/1000g. A latex film was manufactured in the same way as Example 5.

The wound latex film could not be unwound even before heating. The sample for testing the strength was prepared using talc as the powder.

### (Comparative Example 6)

Except that 1 part of aluminum hydroxide used in Example 6 (calculated as anhydride) was added in 100 parts of a latex, a latex film was made in the same manner as Comparative Example 1. The wound film could not be unwound.

### (Example 7)

Using an aqueous solution containing 0.2% aluminum nitrate (as alumina) and 150g calcium nitrate tetrahydrate in 1000g water as external coagulant and concurrently as external crosslinking agent, a latex film was fabricated. Thereafter, the outside surface of the film was dipped in an aqueous solution of zirconyl acetate, 1.5% as ZrO₂, and the leaching and vulcanization treatment were conducted in the same manner as Example 2. The results of strength test on the film are shown Table 1. The sample after the adhesion test could easily be unwound.

### (Example 8)

Using an aqueous solution containing 0.2% zirconyl nitrate (as ZrO₂) and 150g calcium nitrate tetrahydrate in 1000g water as external coagulant and concurrently as external crosslinking agent, a latex film was prepared. Thereafter, the outside surface of the film was dipped in an aqueous solution of polyaluminum choloride (2.5% as alumina), leaching and vulcanization treatments were conducted in the same manner as Example 2. The results of the strength test for the film are shown in Table 1. The sample after the adhesion test could easily be unwound.

### (Example 9)

A latex film was prepared in the same manner as Example 7 except that an aqueous solution of titanium lactate of 1.5% as TiO₂ was used in place of zirconyl acetate. The results of strength test of the film are shown in Table 1. The sample after the adhesion test could easily be unwound.

### (Example 10)

An adhesion test of the latex film was conducted in the same way as Example 4 except that carboxylated SBR (SBR 2570X5 made by Nippon Zeon Co.) was used in place of NBR. The sample after the adhesion test could easily be unwound. Zinc oxide was not incorporated in SBR in the experiment.

### (Example 11)

Except that carboxylated CR latex mentioned below was used in place of NBR, a latex film was prepared in the same manner as in Example 5. The results of the strength test for the films are shown in Table 1. The sample after the adhesion test could easily be unwound.

### Carboxylated CR latex made by Toso Co.

| | |
|---|---|
| GFL-280 | 60 parts |
| LA-502 | 40 parts |

(further containing Zinc flower made by Seido Kagakusha Co., 5 parts)

The pH and viscosity of the formulation latex was 8.9 and 37.8 mPs, respectively.

### (Comparative Example 7)

A film was formed using the carboxylated CR latex used in Example 10 and using calcium nitrate as external coagulant. The film thus formed had strong adhesiveness and was impossible to remove from the dipping former. The latex finds an application as an adhesive.

**Table 1**

| | Thickness of film (mm) | Maximum load stress (kgf/cm²) | Maximum load strain (%) | Adhesion test | Remarks |
|---|---|---|---|---|---|
| Example 1 | 0.11 | 390 | 680 | Possible to unwind | |
| Example 2 | 0.12 | 370 | 695 | Possible to unwind | |
| Example 3 | 0.12 | 370 | 680 | Possible to unwind | |
| Example 4 | 0.12 | 380 | 690 | Possible to unwind | |
| Example 5 | 0.13 | 480 | 790 | Possible to unwind | |
| Example 6 | 0.09 | 450 | 720 | Possible to unwind | |
| Example 7 | 0.11 | 290 | 700 | Possible to unwind | |
| Example 8 | 0.10 | 300 | 720 | Possible to unwind | |
| Example 9 | 0.11 | 310 | 680 | Possible to unwind | |
| Example 11 | 0.12 | 256 | 1040 | Possible to unwind | |
| Example 12 | 0.12 | 340 | 732 | Possible to unwind | Easy to wear |
| Example 13 | 0.11 | 350 | 650 | Possible to unwind | Very easy to wear |
| Reference Example 1 | 0.09 | 30 | 640 | Possible to unwind | Poor strength |
| Comparative Example 1 | 0.12 | 410 | 690 | Impossible to unwind | |
| Comparative Example 2 | 0.03 | Not determined | Not determined | Not tested | Defect of film thickness |
| Comparative Example 3 | 0.04 | Not determined | Not determined | Not tested | Defect of film thickness |
| Comparative Example 5 | 0.08 | 530 | 750 | Impossible to unwind | |
| Comparative Example 6 | 0.07 | 490 | 760 | Impossible to unwind | |

### (Example 12)

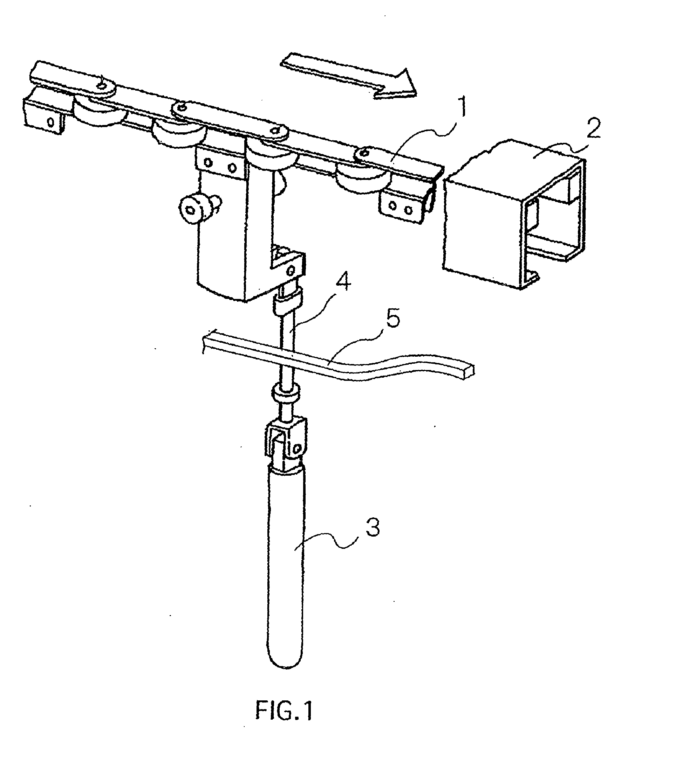

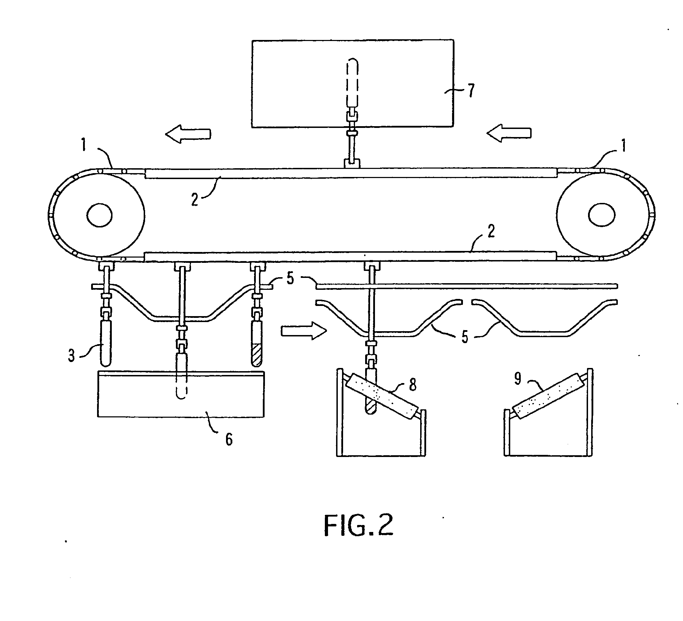

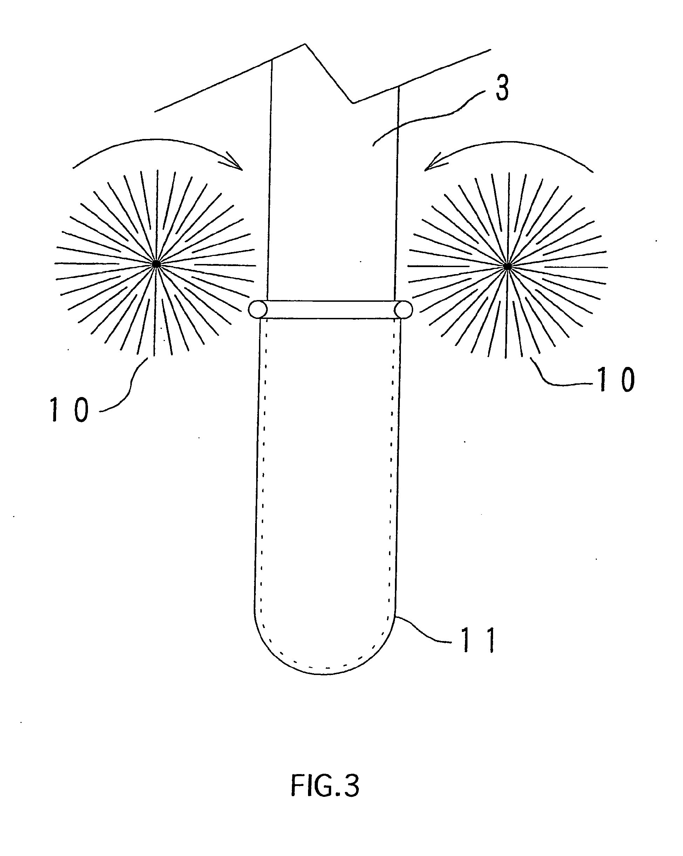

There was fabricated a trial equipment for manufacturing fingerstalls shown in Fig.2 which is equipped with a dipping former transferring apparatus(see Japanese Patent Application Laying-Open (kokai) No.7-329084). Using this equipment, fingerstalls were produced in the same manner as Example 2 to Example 6. The dipping former transferring apparatus of Fig. 1 transfers the dipping former 3 with its chain 1 being moved along the guide rail 2.
The rod 4 moves along the guide 5 and enables the dipping former 3 to move upwards and downwards. In Fig. 2, when the dipping former 3 passes through the dipping tank 6, the dipping former 3 is dipped in the dipping tank with its being lowered. Each of external crosslinking solution tank, external coagulation solution tank, latex liquid tank and leaching treatment tank is made ready previously, the tanks are switched where deemed necessary, and each dipping treatment and leaching treatment are carried out. After each dipping treatment and leaching treatment, the dipping former 3 is transferred to the drying furnace 7 and dried. Except when the winding machine 8 and the unwinding machine 9 are used, the dipping former 3 is kept so that it does not come downwards and kept so that it does not come into contact with the winding machine 8 and the unwinding machine 9. At the time of drying and leaching treatment, the dipping former 3 is stopped to undergo treatment for a predetermined period. The winding machine 8 rotates a roll type brush 10 (Fig.3) previously set up obliquely, and by passing the dipping former through the brush the film 11 affixed on the dipping former 3 is wound. After leaching and subsequent drying treatments, the dipping former 3 is passed through the winding machine 8, and the film deposited onto the dipping former is wound, being removed from the former.

The wound fingerstall was dried at 70 °C for 120 minutes to make the product. This stall was easy to wear on finger. Regarding to the test for film strength, the wound film was immediately unwound, dried at 70 °C for 60 minutes and subjected to the test.

### (Example 13)

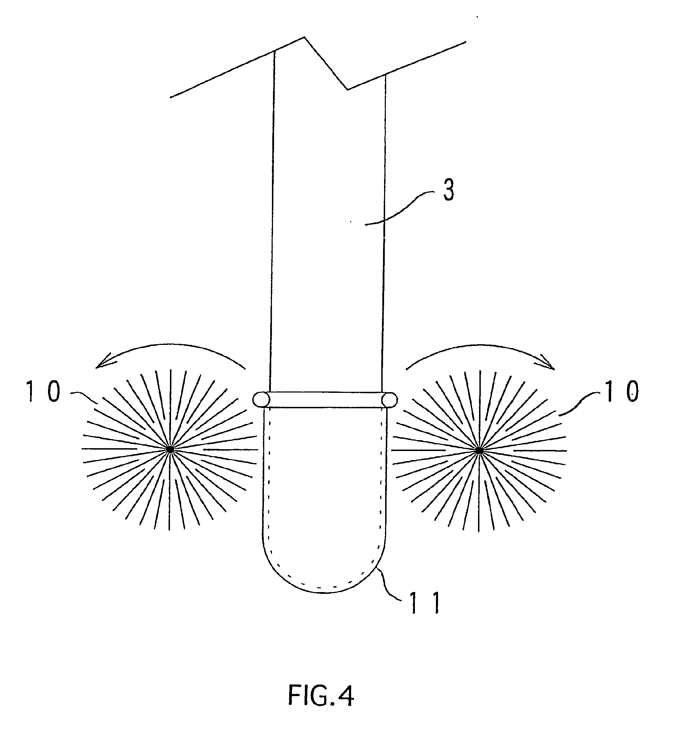

In the same way as in Example 12, the dipping former was passed through the winding machine, and the wound fingerstall was unwound by the unwinding machine 9. The unwinding machine 9, as shown in Fig.4, rotates a roll type brush 10, and when the dipping former 3 is passed there, the machine unwinds the film 11 wound by the winding machine 8. The unwound fingerstall was dried in the drying furnace at 90 °C for 5 minutes. After drying, the stall was again treated on the winding machine 8 and removed from the former to obtain the product.

This method of manufacturing the stall is evaluated to be highly practical, because the drying of the product is easy and the stall is easy to wear on finger because the winding operation is conducted twice. For the film strength test, the films were dried at 90 °C for 5 minutes prior to testing.

### (Example 14)

In the same way as in Example 12 and 13 there were prepared fingerstalls wound by the winding machine 8, but the manufactur conditions were changed as follows. Namely, the dipping former was dipped in the latex liquid 1 cm more deeply than the portion deposited with the coagulant. Further, it was dipped in the external crosslinking agent 2 mm more shallowly than the portion where the coagulant is deposited. When these stalls are unwound, the non-adhesive portions remain as a wound mouth. The unwound fingerstalls became straight fingerstalls with wound mouth and the fingerstalls before unwinding became wound fingerstalls with wound mouth.

### (Example 15)

### (Wear Durability Test)

The fingerstall of a non-adhesive carboxylated latex vulcanized with zinc oxide gave very good strength in the strength test. But it was found that, when it was worn actually, the fingerstall film of the portion which was in contact with finger joints and finger bottoms broke in some cases in several hours. It is considered that expansion and contraction occur during wear because of the ionic crosslinking and that the crosslink points gradually break. This phenomenon is hard to occur in the case of sulfur vulcanization which gives covalent bond.

So, a little slender, ten fingerstalls (16.5 mm in diameter) were worn actually on the middle finger and the medical finger to investigate the number of stalls which break after 24 hours. The results are shown in Table 2. When the emulsion latex is directly added with zinc oxide and the internally added aluminum type inorganic crosslinking agent such as aluminate and aluminum hydroxide gel (0.10 to 0.30 part as Al₂O₃) and when vulcanization is conducted, surprisingly the fingerstalls did not break. Presumably, when the crosslink points of zinc break, aluminum ion makes a crosslink and repairs. The results are shown in Table 2 (also, refer to Table 12.)

The latex film forming conditions were the same as the paragraph mentioned below. As a carboxyl group blocking agent for surface treatment, polyaluminumhydroxide (Paho#2S, an aqueous solution of 0.025% as Al₂O₃) was used, and the vulcanization temperature was 90°C.

The compounds used in examples are as follows:
- Aluminumhydroxide gel: Aluminum hydroxide gel [Tomita] (made by Tomita Seiyaku Co.)
- Sodium aluminate: Sodium aluminate #2019 (made by Asada Kagaku Co.)

- Polyaluminum hydroxide: Paho#2S (made by Asada Kagaku Co.)

**Table 2**

| | | | | | | | |
|---|---|---|---|---|---|---|---|
| Example No. 15 | Content of added NaAlO₂ (as Al₂O₃) (part) | 0 | 0.10 | 0.15 | 0.20 | 0.25 | 0.30 |
| | Number of broken stalls after 24 hour-wearing | 10 | 5 | 2 | 0 | 0 | 0 |
| | | | | | | | |
| | Content of added aluminumhydroxide gel (as Al₂O₃) | 0 | 0.10 | 0.15 | 0.20 | 0.25 | 0.30 |
| | Number of broken stalls after 24 hour-wearing | | | 2 | | 0 | |

### (Screening of Carboxyl Group Blocking Agent)

The following screening was conducted for carboxyl-group blocking agents for surface treatment other than the external crosslinking agents of metal compounds having three or more valences. These compounds generally have low reactivity with carboxyl group at low temperatures. Because of this, these compounds tend to diffuse in the Z-axis direction of the latex film in the steps of dipping and leaching treatments. To reduce the influence of the diffusion of the carboxyl-group blocking agent and to restrain the reduction of the strength, the latex was diluted to half the neat and the concentration of calcium nitrate, an external coagulant, was increased to 300g/1000g from 150g/1000g. Further, as mentioned below, regarding the reaction temperature of the latex film before the strength is expressed, since a higher temperature is desirable for the strength, the temperature of the leaching step was changed to 75 °C. Further, to study the effect of the temperature in the vulcanization step, the vulcanization temperature was set at two levels of 90 and 120 °C. The latex film forming conditions and the adhesion test conditions are as follows, unless otherwise mentioned.

### (Latex Film Forming Conditions)

- Raw material latex:: carboxylated NBR Nipol LX-551
- Vulcanizing agent:: active zinc flower 1.5 parts Sodium aluminate or aluminum hydroxide 0.25 part (as Al₂O₃)
- Solids concentration:: adjusted to 22.5% by diluting with water(dilution, 1:2)
- Coagulant:: calcium nitrate tetrahyrate; 300g/1000g Carboxyl group blocking agent: 0.025% or 0.0025% The dipping former is dipped in the coagulant solution. The amount deposited onto the former is adjusted to 0.03g.
- Dipping:: The dipping former attached with the coagulant is dipped in the latex solution. The dipping former is pulled up after 5 seconds
- Primary drying:: 50 °C, 2 minutes
- Leaching:: 75 °C, 3 minutes
- Drying:: 90 °C, 1 minute
- Outside surface treatment with carboxyl group blocking agent:: The latex film is dipped in the outside surface treatment solution of 0.025% or 0.0025% carboxyl group blocking agent. The amount attached of the surface treatment solution was 0.03g.
- Vulcanization:: The latex film was vulcanized at 90 or 120 °C for 5 minutes. The thickness of the latex film after vulcanization was 0.07 to 0.08 mm, and the weight was 0.3g.

### (Adhesion Test-2)

The latex film prepared under the above-mentioned conditions is vulcanized, wound on the dipping former, and removed from the former as it is. The sample is heated in a hot air dryer of 90 °C for 30 minutes. Then, the sample is taken out from the dryer, cooled and unwound. The mark ○ indicates that unwinding is easy, the mark Δ indicates that unwinding becomes difficult on the way, the mark × indicates that unwinding is considerably difficult and the mark ○' indicates that unwinding is slightly problematic.

### (Example 16 to 18)

To confirm the effect of addition of the aluminum type inorganic crosslinking agent that is internally added in the emulsion latex(aluminum hydroxide gel in Example 16 to 18, sodium aluminate in Example 35 and 61), regarding the aluminum type external crosslinking agent for surface treatment (aluminum nitrate and polyaluminumhydroxide in Example 16, oxazoline compound in Example 17, and carbodiimide compound in Example18) there was studied the effect of the concentration of the carboxyl group blocking agent. Here, the vulcanization temperature of the aluminum type external crosslinking agent (aluminum nitrate or polyaluminum hydroxide) was 90 °C, and that of oxazoline and carbodiimide compounds was 120 °C.

In the case of the aluminum type external crosslinking agent, even when the emulsion latex is not added with the internally added aluminum type inorganic crosslinking agent, the latex product becomes non-adhesive. When the internally added aluminum type inorganic crosslinking agent is used, the concentration of the carboxyl group blocking agent for surface treatment is lowered.

On the other hand, in the case where the carboxyl group blocking agent for surface treatment is a water-soluble oxazoline compound or water-soluble carbodiimide compound, if the internally added aluminum type inorganic crosslinking agent is not added, the effect of the carboxyl-group blocking agent for surface treatment is small. However, although classified as compounds of the same kind, in the case of emulsion type carboxyl group blocking agent for surface treatment, even when the internally added aluminum type inorganic crosslinking agent is not added, the non-adhesion effect is obtained. In the case of water-soluble carboxyl group blocking agent for surface treatment, the carboxyl-group blocking agent diffuses in the Z-axis direction to result in a small effect of the carboxyl-group blocking agent. On the other hand, if the internally added aluminum type inorganic crosslinking agent is added, the diffusion of the carboxyl-group blocking agent is restrained, and thus non-adhesion effect appears. The results are shown in Table 3. The compounds used in this example are as follows.
- Aluminum hydroxide gel:: aluminum hydroxide gel[Tomita] (made by Tomita Seiyaku Co.)
- Polyaluminum hydroxide:: Paho#2S (made by Asada Kagaku Kogyo Co.)
- Oxazoline compound:: Epocross W (aqueous solution) (made by Nihon Shokubai Co.) Epocross K-2030(emulsion) (made by Nihon Shokubai Co.)
- Carbodiimide compound:: Carbodilite E-01(emulsion) (made by Nisshinbo Co.) Carbodilite V-02(aqueous solution) (made by Nisshinbo Co.)

Example 35 used a modified polyamine polyurea resin (Sumirez Resin 712).

This resin is so reactive that it causes the latex to coagulate when added to the latex. In the case of such a compound, as in the aluminum type external crosslinking agent, non-adhesion effect appears in even a system to which the internally added aluminum type inorganic crosslinking agent is not added. Further, alkylketene dimer of Example 61 is an emulsion and is reactive, and, as with the emulsion of Example 17 and 18, even the system in which the internally added aluminum type inorganic crosslinking agent is not added possesses the non-adhesion effect.

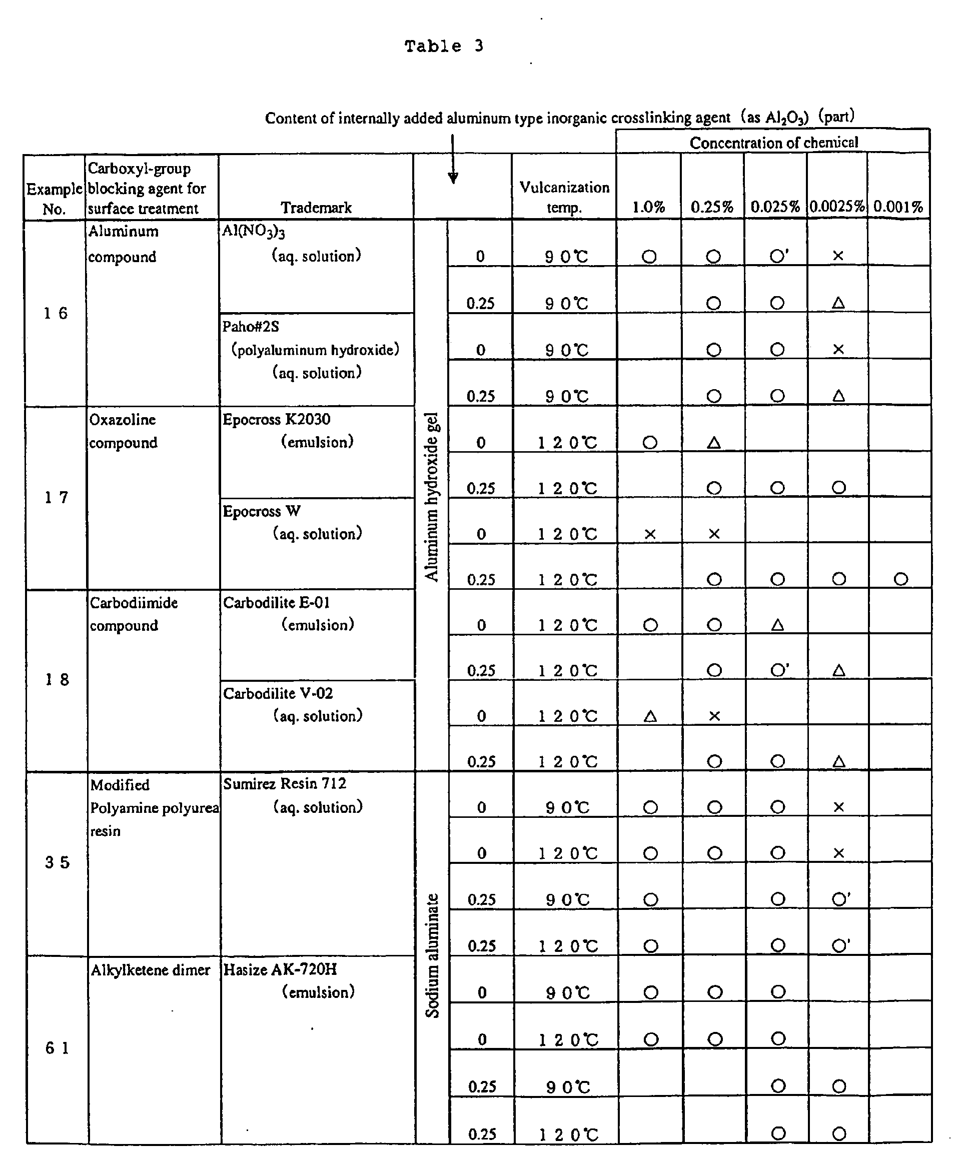

### (Example 19)

Next, to study the influence of the diffusion of the carboxyl group blocking agent for surface treatment, there was studied the effect of a temperature of the leaching step before vulcanization.

As the carboxyl-group blocking agent, the water-soluble oxazoline compound (Epocross W, made by Nippon shokubai Co.) was used. The concentration was varied from 0.1% to 0.001%, and the temperature of the leaching step was varied from 50 to 75 °C. The results are shown in Table 4.

In the case of the treatment concentration as high as 0.1%, when the leaching step temperature is low, the strength becomes low and the non-adhesion effect tends to be low.

It is considered that, even in the case that the internally added aluminum type inorganic crosslinking agent (sodium aluminate, 0.25 part as Al₂O₃) was added, there appeared the influence of the diffusion in low temperatures.

**Table 4**

| | | | | Temp. of leaching step | | | | | |
|---|---|---|---|---|---|---|---|---|---|
| Example No. | Carboxyl-group crosslinking organic agent for surface treatment | Conc. | | 50°C | 55°C | 60°C | 65°C | 70°C | 75°C |
| 19 | Oxazoline compound Epocross W | 0.1% | Adhesion test | × | ○ | ○ | ○ | | ○ |
| | | | Strength (Mpa) | | 27.9 | 31.8 | 29.8 | 30.5 | 37.7 |
| | | 0.025% | Adhesion test | × | ○ | ○ | ○ | ○ | ○ |
| | | | Strength (Mpa) | | | | | | 37.3 |
| | | 0.005% | Adhesion test | | | | | | |
| | | | Strength (Mpa) | | | | | | 35.1 |
| | | 0.001% | Adhesion test | × | | | | | ○ |
| | | | Strength (Mpa) | | | | | | 35.1 |

### (Example 20 to 27)

Regarding to the compounds considered as crosslinking agents for carboxyl group, there was studied how the concentration of the carboxyl group blocking agent for surface treatment and the vulcanization temperature (90 and 120 °C) had effect on the non-adhesion in the system in which the internally added aluminum type inorganic crosslinking agent was added. The test results are shown in Table 5.

Carboxyl group blocking agents for surface treatment (i.e., organic crosslinking agents for carboxyl group):
(Example 20) polyamine triethylenetetramine(made by Wako Seiyaku)
(Example 21) melamine resin, Sumitex Resin M-3 (made by Sumitomo Kagaku Kogyo)
(Example 22) melamine resin, Sumirez Resin 613special (made by Sumitomo Kagaku Kogyo)
(Example 23) amino group-containing urethane resin, Superflex R-3000 (made by Daiichi Kagaku Kogyo)
(Example 24) blocked isocyanate prominate, XC-915 (made by Takeda Yakuhin Kogyo)
(Example 25) multifunctional epoxy compound, Denacol EX-614B (made by Nagase Kasei Kogyo)
(Example 26) epoxycresolnovolac resin emulsion, Denacol EM-150 (made by Nagase Kasei Kogyo)
(Example 27) bifunctional epoxy compound, Denacol EX-313 (made by Nagase Kasei Kogyo)

**Table 5**

| | | | | Conc. of organic crosslinking agent | | | |
|---|---|---|---|---|---|---|---|
| Example No. | Carboxyl-group crosslinking organic agent for surface treatment | Trademark | Vulcanization temp. | 0.025% | 0.01% | 0.005% | 0.0025% |
| 20 | Polyamine | Triethyltetramine | 90°C | ○ | | | |
| | | | 120°C | Δ | | | |
| 21 | Melamine resin | Sumitex Resin M-3 | 90°C | ○ | | | |
| | | | 120°C | Δ | | | |
| 22 | Melamine resin | Sumitex Resin 613special | 90°C | ○ | | ○ | |
| | | | 120°C | ○ | | | |
| 23 | Amino group-containing urethane resin | Superflex R-3000 (urethane) | 90°C | ○ | | ○ | |
| | | | 120°C | ○ | | | |
| 24 | Blocked isocyanate | Prominate XC-915 | 90°C | ○ | | | |
| | | | 120°C | ○ | | | |
| 25 | Polyfunctional epoxy compound | Denacol EX-614B | 90°C | Δ | ○ | ○' | |
| | | | 120°C | ○ | ○ | | |
| 26 | Epoxycresolnovolac resin | Denacol EM-150 | 90°C | ○ | | | ○ |
| | | | 120°C | ○ | | | ○ |
| 27 | Bifunctional epoxy compound | Denacol EX-313 | 90°C | ○ | | ○ | |
| | | | 120°C | ○ | | | |

### (Example 28 to 36)

In the same manner as in Example 20 to 27, regarding the hydrogen bond formation regulators used in the field of paper, the test was conducted to evaluate their performance as carboxyl group blocking agent. These compounds have been developed as waterproof endowing agent, printability improver, wet paper strength reinforcing agent and the like of papers, with their workability and safety taken into consideration. The results are shown in Table 6. As in the case of the organic crosslinking agent for carboxyl group, the compounds give the non-adhesion effect.

As to Example 35, the test was conducted for the system in which the internally added aluminum type inorganic crosslinking agent, sodium aluminate, was not added.

Carboxyl group blocking agent for surface treatment (i.e., hydrogen bond formation regulators):
(Example 28) glyoxal (made by Wako Junyaku Co.)
(Example 29) polyamide resin, Sumirez Resin 5001 (made by Sumitomo Kagaku Kogyo Co.)
(Example 30) polyamide resin, Sunmide X-13A (made by Sanwa Kagaku Kogyo Co.)
(Example 31) polyamidepolyurea resin, Sumirez Resin 636 (made by Sumitomo Kagaku Kogyo Co.)
(Example 32) polyamideepoxy resin, Sumirez Resin 675 (made by Sumitomo Kagaku Kogyo Co.)
(Example 33) polyaminepolyurea resin, Sumirez Resin 302 (made by Sumitomo Kagaku Kogyo Co.)
(Example 34) polyaminepolyurea resin, PA-620 (made by Nippon PMC)
(Example 35) modified polyaminepolyurea resin, Sumirez Resin 712 (made by Sumitomo Kagaku Kogyo Co.)
(Example 36) polyamidepolyureaglyoxal condensation reaction product, Sumirez Resin 5004 (made by Sumitomo Kagaku Kogyo Co.)

**Table 6**

| | | | | | Conc. of reactive organic compound | | | |
|---|---|---|---|---|---|---|---|---|
| Example No. | Hydrogen bond formation regulator for surface treatment | Trademark | | Vulcanization temp. | 0.025% | 0.01% | 0. 005% | 0. 0025% |
| 28 | Glyoxal | | | 90°C | Δ | | | |
| | | | | 120°C | ○ | | | |
| 29 | Polyamide resin | Sumirez Resin 5001 | | 90°C | ○ | | ○ | ○ |
| | | | | 120°C | ○ | | | ○ |
| 30 | Polyamide resin | Sunmide X-13A | | 90°C | ○ | | | |
| | | | | 120°C | ○ | | | |
| 31 | Polyamide polyurea resin | Sumirez Resin 636 | | 90°C | ○ | | | ○ |
| | | | | 120°C | ○ | | | ○ |
| 32 | Polyamide epoxy resin | Sumirez Resin 675 | | 90°C | ○' | | | |
| | | | | 120°C | ○ | | | |
| 33 | Polyamine polyurea resin | Sumirez Resin 302 | | 90°C | ○ | | | ○' |
| | | | | 120°C | ○ | | | ○ |
| 34 | Polyamine polyurea resin | PA-620 | | 90°C | ○ | | | |
| | | | | 120°C | ○ | | | |
| 35 | Modified polyamine polyurea resin | Sumirez Resin 712 | | 90°C | ○ | | ○ | ○' |
| | | | | 120°C | ○ | | | ○' |
| | | | NaAlO₂ | 90°C | ○ | | | × |
| | | | not added | 120°C | ○ | | | × |
| 36 | Polyamidepolyureaglyoxal condensation reaction product | Sumirez Resin 5004 | | 90°C | ○ | ○' | ○ | |
| | | | | 120°C | ○ | | | |

### (Example 37 to 39)

The test was conducted in the same manner as in Example 20 to 34 concerning the monofunctional carboxyl group blocking agent. The results are shown in Table 7. The agent, which is monofunctional, cannot crosslink a carboxyl group, and yet it has the non-adhesion effect. It is noticed that blocking a carboxyl group and forming hydrophobicity are both important for the non-adhesion of the latex product.

Surface carboxyl blocking agents for surface treatment (i.e., monofunctional carboxyl blocking agents):
(Example 37) monofunctional modified bisphenol A type epoxy emulsion, Denacast EM-101 (made by Nagase Kasei Kogyo Co.)
(Example 38) monofunctional modified bisphenol A type epoxy emulsion, Denacast EM-103 (made by Nagse Kase Kogyo Co.)
(Example 39) monofunctional epoxy, Denacol EX-145 (made by Nagase Kasei Kogyo Co.)

**Table 7**

| | | | | Conc. of monofunctional carboxyl-group blocking agent | | | |
|---|---|---|---|---|---|---|---|
| Example No. | Monofunctional carboxyl-group blocking agent for surface treatment | Trademark | Vulcanization temp. | 0.025% | 0.01% | 0.005% | 0.0025% |
| 37 | Monofunctional epoxy | Denacast EM-101 (emulsion) | 90°C | ○ | | | |
| | | | 120°C | ○ | | | |
| 38 | Monofunctional epoxy | Denacast EM-103 (emulsion) | 90°C | ○ | | ○ | ○' |
| | | | 120°C | ○ | | | ○ |
| 39 | Monofunctional epoxy | Denacol EX-145 | 90°C | ○ | | | |
| | | | 120°C | ○ | | | |

### (Example 40 to 58)

In the same manner as in Example 20 to 39, the non-adhesion surfactants (cationic, amphoteric, nonionic, or anionic) were tested for their performance as carboxyl-group blocking agents, including a test for the effect of adding an internally added aluminum type inorganic crosslinking agent, sodium aluminate. The results are shown in Table 8. In the system in which sodium aluminate was not added, the non-adhesion effect of the surfactant is small.

Carboxyl group blocking agent for surface treatment (non-adhesive surfactant):
Non-adhesion cationic surfactant:
   (Example 40) quarternary amine, Quartermin 86W (made by Kao Co.)
   (Example 41) quarternary amine, Catinal MB 50A (made by Toho Kagaku Kogyo)
   (Example 42) imidazoline type betaine, Anhitol 20YB (made by Kao)
   (Example 43) oxide type betaine, Softamin L (made by Toho Kagaku Kogyo)
   (Example 44) alkylamide type betaine, Ovazolin CAB-30 (made by Toho Kagaku Kogyo)
Non-adhesive nonionic surfactant:
   (Example 45) tertiary amine, Esomin C/12 (made by Lion)
   (Example 46) tertiary alkylamine, Amito 105 (made by Kao)
   (Example 47) alkanolamide, Aminon PK-02S (made by Kao)
   (Example 48) polyoxyethylenelauryether, Emulgen 109P (made by Kao)
   (Example 49) polyoxyethylene derivative, Emulgen A60 (made by Kao)
Non-adhesive anionic surfactant:
   (Example 50) β-naphthalenesulfonic acid formalin condensation product, Demol N (made by Kao)
   (Example 51) alkylmethyltaurinate, Lipotac TE (made by Lion)
   (Example 52) disodium dodecyldiphenylethersulfonic acid, Perex SS-L (made by Kao)

**Table 8**

| | | | | | | Conc. of surfactant | | | |
|---|---|---|---|---|---|---|---|---|---|
| Example No. | | Non-adhesive surfactant for surface treatment | Trademard | | Vulcanization temp. | 0.025% | 0.01% | 0.005% | 0.0025% |
| 40 | Cation | Quarternary amine | Quatermin 86W | | 90°C | ○ | | | |
| | | | | | 120°C | ○ | | | |
| | | | | NaAlO₂ | 90°C | × | | | |
| | | | | not added | 120°C | × | | | |
| 41 | Cation | Quarternary amine | Catinal MB-50A | | 90°C | ○' | | | ○' |
| | | | | | 120°C | ○ | | | ○ |
| | | | | NaAlO₂ | 90°C | × | | | × |
| | | | | not added | 120°C | × | | | × |
| 42 | Amphoteric | Imidazoline type betaine | Anhitol 20YB | | 90°C | ○ | ○' | Δ | |
| | | | | | 120°C | ○ | | | |
| | | | | NaAlO₂ | 90°C | × | × | × | × |
| | | | | not added | 120°C | × | | | Δ |
| 43 | Amphoteric | Oxide type betaine | Softamin L | | 90°C | Δ | | | ○ |
| | | | | | 120°C | ○ | | | ○ |
| | | | | NaAlO₂ | 90°C | × | | | × |
| | | | | not added | 120°C | × | | | × |
| 44 | Amphoteric | Alkylamide type betaine | Ovazolin CAB-30 | | 90°C | ○ | | ○ | |
| | | | | | 120°C | ○ | | | |
| | | | | NaAlO₂ | 90°C | × | | × | |
| | | | | not added | 120°C | × | | | |
| 45 | Nonion | Tertiary alkylamine | Esomin C/12 | | 90°C | ○' | | | ○' |
| | | | | | 120°C | ○ | | | ○ |
| | | | | NaAlO₂ | 90°C | × | | | × |
| | | | | not added | 120°C | × | | | × |
| 46 | Nonion | Tertiary alkylamine | Amito 105 | | 90°C | Δ | | | |
| | | | | | 120°C | ○ | | | |
| | | | | NaAlO₂ | 90°C | × | | | |
| | | | | not added | 120°C | × | | | |
| 47 | Nonion | Alkanolamide (lauryldiethanolamide) | Aminon PK-02S | | 90°C | ○ | ○ | Δ | |
| | | | | | 120°C | ○ | | | |
| | | | | NaAlO₂ | 90°C | × | × | × | |
| | | | | not added | 120°C | × | | | |
| 48 | Nonion | Polyoxyethylene laurylether | Emulgen 109P | | 90°C | Δ | | | |
| | | | | | 120°C | ○ | | | |
| | | | | NaAlO₂ | 90°C | × | | | |
| | | | | not added | 120°C | × | | | |
| 49 | Nonion | Polyoxyethylene derivative | Emulgen A60 | | 90°C | Δ | | | |
| | | | | | 120°C | ○ | | | |
| | | | | NaAlO₂ | 90°C | × | | | |
| | | | | not added | 120°C | × | | | |
| 50 | Anion | β -naphthalenesulfonic acid formalin condensation product | Demol N | | 90°C | ○ | | | ○' |
| | | | | | 120°C | ○ | | | ○ |
| | | | | NaAlO₂ | 90°C | × | | | × |
| | | | | not added | 120°C | × | | | × |
| 51 | Anion | Alkylmethyltaurinate | Lipotac TE | | 90°C | ○' | | | Δ |
| | | | | | 120°C | ○ | | | |
| | | | | NaAlO₂ | 90°C | × | | | × |
| | | | | not added | 120 °C | × | | | × |
| 52 | Anion | Disodium dodecyldiphenylethersulfonate | Perex SS-L | | 90°C | Δ | | | |
| | | | | | 120°C | ○' | | | |
| | | | | NaAlO₂ | 90°C | × | | | |
| | | | | not added | 120°C | × | | | |

### (Example 53 to 58)

Regarding the polymer type derivatives, to evaluate their performance as carboxyl group blocking agent, the test was conducted in the same manner as in Example 40 to 52. Although these compounds are sometimes treated as polymer surfactant, they are employed as non-adhesive polymer type surfactants in the present invention. The results are shown in Table 9.

Carboxyl group blocking agent for surface treatment (i.e., non-adhesive polymer type surfactants):
(Example 53) cellulose derivative, Reoguard KGP (made by Lion)
(Example 54) cationized starch, CATO 308 (made by Nippon NSC)
(Example 55) cationized starch, Opti Bond 3282 (made by Nippon NSC)
(Example 56) dimethyldiallyammoniumchloride acrylamide copolymer, ME polymer 09W (made by Toho Kagaku Kogyo)
(Example 57) cationic polyurethane dispersion in water, F-8570D (made by Daiichi Kogyo Seiyaku)
(Example 58) ammonium polystyrenesulfonate, VERSA-TLYE915 (made by Nippon NSC)

**Table 9**

| | | | | | | Conc. of polymer surfactant | | | |
|---|---|---|---|---|---|---|---|---|---|
| Example No. | | Non-adhesive polymer surfactant for surface treatment | Trademark | | Vulcanization temp. | 0.025% | 0.01% | 0. 005% | 0.0025% |
| 53 | Cation | Cellulose derivative | Reoguard KGP | | 90°C | Δ | | | |
| | | | | | 120°C | ○ | | | |
| | | | | NaAlO₂ | 90°C | × | | | |
| | | | | not added | 120°C | × | | | |
| 54 | Cation | Cationized starch | CATO 308 | | 90°C | ○ | | | Δ |
| | | | | | 120°C | ○ | | | ○' |
| | | | | NaAlO₂ | 90°C | × | | | |
| | | | | not added | 120°C | × | | | |
| 55 | Cation | Cationized starch | Opti Bond 3282 | | 90°C | ○' | | | |
| | | | | | 120°C | ○ | | | |
| | | | | NaAlO₂ | 90°C | × | | | |
| | | | | not added | 120°C | × | | | |
| 56 | Cation | Dimethyldiallylammonium chloride acrylamide copolymer | ME polymer 09W | | 90°C | ○' | | | |
| | | | | | 120°C | ○ | | | |
| | | | | NaAlO₂ | 90°C | × | | | |
| | | | | not added | 120°C | × | | | |
| 57 | Cation | Cationic polyurethane dispersion in water | F-8570D | | 90°C | ○ | | | |
| | | | | | 120°C | | | | |
| | | | | NaAlO₂ | 90°C | × | | | |
| | | | | not added | 120°C | | | | |
| 58 | Anion | Ammonium polystrenesulforate | VERSA-TLYE915 | | 90°C | Δ | | | |
| | | | | | 120°C | ○ | | | |
| | | | | NaAlO₂ | 90°C | × | | | |
| | | | | not added | 120°C | × | | | |

### (Example 59 to 64)

Regarding paper sizing agents, to evaluate their performance as carboxyl group blocking agent, the test was conducted in the same way as in Example 20 to 39. The results are shown in Table 10. Concerning Example 61, the test was conducted for the system in which the internally added aluminum type inorganic crosslinking agent, sodium aluminate, was not added.

Carboxyl group blocking agent for surface treatment (i.e., sizing agent):
(Example 59) fortified rosin, Sizepine E-50 (made by Arakawa Kagaku Kogyo)
   This sizing agent coagulates with a calcium salt. So, the treatment of the inside surface was conducted with Sizepine N-773 (concentration: 0.0025%) mentioned below, and the treatment of the outside surface was conducted with Sizepine E.
(Example 60) emulsion type rosin sizing agent, Sizepine N-773 (made by Arakawa Kagaku Kogyo)
(Example 61) alkylketene dimer, Hasize AK-720H (made by Harima Kasei)
(Example 62) styrene type synthetic sizing agent, BLS-720 (made by Misawa Ceramics)
(Example 63) olefin type synthetic sizing agent, Hamacoat AK-505 (made by Misawa Ceramics)
(Example 64) alkenyl succinate, Sizepine (made by Arakawa Kagaku Kogyo)

**Table 10**

| | | | | | Conc. of sizing agent | |
|---|---|---|---|---|---|---|
| Example No. | Sizing agent for surface treatment | Trademark | | Vulcanization temp. | 0.025% | 0.0025% |
| 59 | Fortified rosin sizing agent | Sizepine E-50 | | 90 °C | ○ | |
| | | | | 120 °C | ○ | |
| 60 | Emulsion type rosin sizing agent | Sizepine N-773 | | 90 °C | ○ | ○ |
| | | | | 120 °C | ○ | ○ |
| 61 | Alkylketene dimer | Hasize AK-720H | | 90 °C | ○ | ○ |
| | | | | 120 °C | ○ | ○ |
| | | | NaAlO₂ | 90 °C | ○ | |
| | | | not added | 120 °C | ○ | |
| 62 | Styrene type synthetic sizing agent | BLS-720 | | 90 °C | ○ | ○ |
| | | | | 120 °C | ○ | ○ |
| 63 | Olefin type synthetic sizing agent | Hamacoat AK-505 | | 90 °C | ○ | ○' |
| | | | | 120 °C | ○ | ○ |
| 64 | Alkenyl succinate | Sizepine S-400S | | 90 °C | ○ | ○ |
| | | | | 120 °C | ○ | ○ |

### (Example 65 to 81)

The performance test was conducted for carboxyl group blocking agents in the same manner as in Example 20 to 64. The carboxyl-group blocking agents were directly added to confirm their effect. Accordingly, the latex film was immediately vulcanized after the leaching treatment to make each sample. The adhesion test was conducted according to the adhesion test 2. The results are shown in Table 11. It is noticed that any type of carboxyl group blocking agents for surface treatment is effective as an internally added carboxyl-group blocking agent. However, such a carboxyl-group blocking agent that coagulates the latex when it is incorporated into a latex emulsion is not desirable. In such a case it is necessary to take a measure such as stabilizing the latex emulsion by adding a surfactant. Also studied was the influence of addition of an internally added aluminum type inorganic crosslinking agent, sodium aluminate. In the case of a reactive carboxyl-group blocking agent (in Example 70 and 77) and a carboxyl-group blocking agent which forms an insoluble salt with calcium ion (in Example 76, 78 and 81), the non-adhesion effect appears even in the system in which sodium aluminate is not added. When a polyamide polyurea resin (Sumirez Resin 703) is added, the latex emulsion is coagulated without addition of sodium aluminate, but with addition of it, the sodium aluminate disperses Sumirez Resin 703 homogeneously. Thus, without coagulation of the emulsified latex, the non-adhesion effect is observed.

Internally added carboxyl group blocking agents:
(Example 65) monofunctional modified bisphenol A type epoxy emulsion, Denacast EM-103 (made by Nagase Kasei Kogyo)
(Example 66) carbodiimide crosslinking agent, Carbodilite V-20 (made by Toyobo)
(Example 67) oxazoline crosslinking agent, Epocross W (made by Nihon Shokubai)
(Example 68) self-emulsifying type polyisocyanate, Aquanate 200 (made by Nihon Polyurethane Kogyo)
(Example 69) blocked isocyanate, Prominate XC-915 (made by Takeda Seiyaku)
(Example 70) polyamide resin, Sumirez Resin 5001 (made by Sumitomo Kagaku Kogyo)
(Example 71) polyamidepolyurea resin, Sumirez Resin 703 (made by Sumitomo Kagaku Kogyo)
(Example72) β-naphthalenesulfonic acid formalin condensation producct, Demol N (made by Kao)
(Example 73) alkylnaphthalenesulfonic acid formalin condensation polymerization product, Polyty N-100 (made by Lion)
(Example 74) alkylmethyltaurinate, Lipotac TE made by Lion)
(Example 75) emulsion type modified rosin sizing agent, Halfsize NES-650 (made by Harima Kasei)
(Example 76) fortified rosin sizing agent, Sizepine (made by Arakawa Kagaku Kogyo)
(Example 77) alkylketene dimer, Halfsize AK-720H (made by Harima Kasei)
(Example 78) alkenylsuccinate, Sizepine S-400S (made by Arakawa Kagaku Kogyo)
(Example 79) alkenylsuccinic acid anhydride, Colopearl Z-100S (made by Seiko Kagaku Kogyo)
(Example 80) styrene acryl type surface sizing agent, Colopearl M-150-2 (made by Seiko Kagaku Kogyo)
(Example 81) saponification product of an adduct of branched olefin and maleic anhydride, RFsize NSP-SH (made by Seiko Kagaku Kogyo)

**Table 11**

| | | | | | Amount added (part) | |
|---|---|---|---|---|---|---|
| Example No. | Internally added carboxyl-group blocking agent | Trademark | | Vulcanization temp. | 2.5 | 1.0 |
| 65 | Monofunctional epoxy | Denacast EM-103 | | 90°C | ○' | × |
| | | | | 120°C | ○' | ○ |
| | | | NaAlO₂ | 90°C | × | |
| | | | not added | 120°C | × | |
| 66 | Carbodiimide | Carbodilite V-02 | | 90°C | ○' | ○' |
| | | | | 120°C | ○' | ○ ○ |
| | | | NaAlO₂ | 90°C | × | |
| | | | not added | 120°C | × | |
| 67 | Oxazoline | Epocross W | | 90°C | ○ | ○' |
| | | | | 120°C | ○ | ○ |
| | | | NaAlO₂ | 90°C | × | |
| | | | not added | 120°C | × | |
| 68 | Self-emulsifying polyisocyanate | Aquanate 200 | | 90°C | | ○' |
| | | | | 120°C | | ○ |
| | | | NaAlO₂ | 90°C | × | |
| | | | not added | 120°C | × | |
| 69 | Blocked isocyanate | Prominate XC-915 | | 90°C | | ○ |
| | | | | 120°C | | ○ |
| | | | NaAlO₂ | 90 °C | × | |
| | | | not added | 120°C | Δ | |
| 70 | Polyamide resin | Sumirez Resin 5001 | | 90°C | ○ | ○ |
| | | | | 120°C | ○ | ○ |
| | | | NaAlO₂ | 90°C | Δ | |
| | | | not added | 120°C | ○' | |
| 71 | Polyamide polyurea resin | Sumirez Resin 703 | | 90°C | ○ | ○' |
| | | | | 120°C | ○ | ○ |
| | | | NaAlO₂ | 90°C | aggregate | |
| | | | not added | 120°C | | |
| 72 | β -naphthalenesulfonic acid formalin condensate | Demol N | | 90°C | ○ | ○' |
| | | | | 120°C | ○ | ○ |
| | | | NaAlO₂ | 90°C | × | |
| | | | not added | 120°C | Δ | |
| 73 | Alkylnaphthalenesulfonic acid formalin condensate | Polyty N-100K | | 90°C | | Δ |
| | | | | 120°C | | ○ |
| | | | NaAlO₂ | 90°C | × | |
| | | | not added | 120°C | × | |
| 74 | Alkylmethyltaurinate | Lipotac TE | | 90°C | ○ | × |
| | | | | 120°C | ○ | ○ |
| | | | NaAlO₂ | 90°C | × | |
| | | | not added | 120°C | Δ | |
| 75 | Emulsion type rosin sizing agent (modified rosin) | Hasize NES-650 | | 90°C | ○' | Δ |
| | | | | 120°C | ○ | ○ |
| | | | NaAlO₂ | 90°C | × | |
| | | | not added | 120°C | Δ | |
| 76 | Fortified rosin sizing agent | Sizepine E-50 | | 90°C | ○ | × |
| | | | | 120°C | ○ | ○ |
| | | | NaAlO₂ | 90°C | Δ | |
| | | | not added | 120°C | ○' | |
| 77 | Alkylketene dimer | Hasize AK-720H | | 90°C | ○ | ○ |
| | | | | 120°C | ○ | ○ |
| | | | NaAlO₂ | 90°C | × | |
| | | | not added | 120°C | ○' | |
| 78 | Alkenyl succinate | Sizepine S-400S | | 90°C | ○ | ○' |
| | | | | 120°C | ○ | ○ |
| | | | NaAlO₂ | 90°C | ○' | |
| | | | not added | 120°C | ○ | |
| 79 | Alkenyl succinic acid anhydride | Coropearl Z-100S | | 90°C | ○ | ○' |
| | | | | 120°C | ○ | ○ |
| | | | NaAlO₂ | 90°C | × | |
| | | | not added | 120°C | Δ | |
| 80 | Styrene-acryl type surface sizing agent | Coropearl M-150-2 | | 90°C | ○ | Δ |
| | | | | 120°C | ○ | ○ |
| | | | NaAlO₂ | 90°C | × | |
| | | | not added | 120°C | Δ | |
| 81 | Branched olefin-maleic anhydride adduct | RF size NSP-SH | | 90°C | | Δ |
| | | | | 120°C | | ○ |
| | | | NaAlO₂ | 90°C | ○' | |
| | | | not added | 120°C | ○ | |

### (Example 82 to 85)

Fingerstalls were prepared in the same way as in Example 29 except that internally added aluminum type inorganic crosslinking agents as described below were used in place of sodium aluminate, and the adhesion and wear durability tests were conducted. The amount of the internally added aluminum type inorganic crosslinking agent was 0.25 part as Al₂O₃. The carboxyl-group blocking agent used was polyamide resin (Sumirez Resin 5001). The concentration of the polyamide resin for surface treatment was 0.025%. The results are shown in Table 12. As in the case of Example 29, the latex film was non-adhesive. Any fingerstall did not break in the wear durability test, thus passed the test.
(Example 82) calcium aluminate (made by Soekawa Rikagaku)
(Example 83) magnesium aluminate (made by Soekawa Rikagaku)
(Example 84) aluminum magnesium hydroxide, Aluminum magnesium hydroxide 251 (made by Tomita Seiyaku)
(Example 85) synthetic hydrotalcite, Synthetic hydrotalcite H (made by Tomita Seiyaku)

**Table 12**

| Example No. | Internally added aluminum type inorganic crosslinking agent | Trademark | Vulcanization temp. | Adhesion test 2 | Number of breakings |
|---|---|---|---|---|---|
| 82 | Calcium aluminate | | 90°C | Δ | 0 |
| | | | 120°C | ○ | 0 |
| 83 | Magnesium aluminate | | 90°C | Δ | 0 |
| | | | 120°C | ○ | 0 |
| 84 | Aluminum · magnesium hydroxide | Aluminum · magnesium hydroxide 251 | 90°C | ○ | 0 |
| | | | 120°C | ○ | 0 |
| 85 | Synthetic hydrotalcite | Synthetic hydrotalcite | 90°C | ○' | 0 |
| | | | 120°C | ○ | 0 |

### (Example 86)

In manufacturing fingerstalls in the same way as in Example 2 and 3, the dipping former was dipped and deposited with the external crosslinking agent for the inside surface, dried, and dipped in the methanolic external coagulant liquid 1 cm more deeply than the portion of said crosslinking agent and dried. Then the former was dipped in the latex slightly more shallowly than the part of the methanolic external coagulation liquid, and further it was dipped in the external crosslinking liquid for the outside surface in the same depth as the external crosslinking agent for the inside surface. When these stalls are wound and then unwound, the non-adhesive portions remain as wound mouth. In this case, since the latex is not directly brought into contact with the dipping former and since the thickness of the latex is increased due to the coagulation liquid, the removal of the latex film from the dipping former is easy and the winding is also easy.

### (Example 87)

In forming fingerstalls in the same way as in Example 14 and 86, the wound mouth was formed after the former was dipped in the latex. By coating an external crosslinking liquid for the outer surface, non-adhesive fingerstalls with wound mouth could be formed. In this case, even if the former is immersed in the external crosslinking liquid more deeply than the wound mouth portion, the wound mouth does not unwind and so it is not necessary to mind the depth of the external crosslinking liquid on the outer surface.

### (Example 88)

In forming fingerstalls in the same way as in Example 4, the former was immersed in the latex more shallowly than the external coagulation liquid and the external crosslinking liquid to form a wound mouth. As in Example 86, since the latex has an increased thickness owing to the coagulation liquid, the removal of the latex-formed film from the dipping former is easy and making the bottom windings is also easy. In this case, if the latex is too dry when a wound mouth is formed, the wound mouth unwinds . But, if the wound mouth is made when the latex is half gelled, and if it is dried it does not unwind.

### (Example 89)

Fingerstalls were formed using the trial-use fingerstall manufacturing equipment (Fig.2) under the same latex film forming conditions as in the said screening of carboxyl-group blocking agents. The former was immersed in the coagulation liquid and dried, and thereafter immersed in the latex incorporated with 1.5 parts of zinc oxide, 0.25 part (as Al₂O₃) of sodium aluminate and 1.5 parts of zinc oxide, and then dried. After subjected to the treatments of mouth-winding, leaching and drying, the former was immersed in the outside surface treating agent of 0.025% polyamide resin, Sumirez resin 5001 (made by Sumitomo Kagaku Kogyo) and dried at 90 °C for 2 minutes. Thereafter, winding and unwinding were successively conducted, and, the film was dried at 90 °C for 3 minutes, again wound on the winding machine and removed from the former in the wound state. In the case of the inorganic outside-surface treating agent, the leaching treatment is necessary after immersion into the outside-surface treating agent. Since this outside-surface treating agent is organic, the leaching treatment is unnecessary after the immersion into the outside-surface treating agent. Hence, the manufacture steps can be reduced.

The stalls removed from the former were dried at 70 °C for 120 minutes to give the final products. The formed stalls were easy to wear on finger.

### (Example 90)

Fingerstalls were formed in the same way as in Example 86 except that the latex was incorporated with 1 part of alkylketene dimer, Halfsize AK-720H (made by Harima Kasei) as carboxyl-group blocking agent. In this case, since the carboxyl-group blocking agent is added to the latex, it is not necessary to deposite the outside-surface treating agent after dipping the former into the latex. Hence, the manufacture steps can be reduced. The manufacture steps are as follows. The former was dipped in the coagulation liquid and dried, then dipped in the latex and dried, subjected to a leaching treatment after winding the mouth, and dried at 90 °C at 2 minutes. Thereafter, winding and unwinding were successively conducted, and the film was dried at 90 °C for 3 minutes. It was again wound on the winding machine and removed from the former in the wound state.

The fingerstalls removed from the former was dried at 70°C for 120 minutes to give the final producta. The stalls were easy to wear on finger.

### [Industrial Applicability]

The present invention provides a powder-free and non-adhesive latex dipped product by adding a carboxyl-group blocking agent into a carboxylated latex, or alternatively by providing a layer treated with the carboxyl-group blocking agent to one or both surfaces of the carboxylated latex product. The non-adhesive latex product have excellent durability since the carboxylated latex contains an internally added aluminum type inorganic crosslinking agent, such as aluminate or aluminum hydroxide gel. In case of a fingerstall, a non-powdered machine-wound product can be prepared on-machine utilizing non-adhesion of the product.

## Claims

1. A non-adhesive carboxylated latex dipped product having a layer on one or both surfaces treated with a carboxyl-group blocking agent, wherein the carboxylated latex is internally added with aluminate or aluminum hydroxide gel.

2. The non-adhesive carboxylated latex dipped product according to claim 1, wherein the carboxyl-group blocking agent is a metal ion crosslinking agent having three or more valences.

3. The non-adhesive carboxylated latex dipped product according to claim 2, wherein the metal ion crosslinking agent is an aluminium compound, titanium compound, zirconium compound or any combination thereof.

4. The non-adhesive carboxylated latex dipped product according to claim 1, wherein the carboxyl-group blocking agent is an organic crosslinking agent for carboxyl groups of carboxylated latex.

5. The non-adhesive carboxylated latex dipped product according to claim 4, wherein the organic crosslinking agent comprises an aziridine compound, an epoxy compound, a blocked isocyanate, an oxazoline compound, a carbodiimido compound, a melamineformaldehyde resin, ureaformaldehyde resins, isocyanates, phenolformaldehyde resins, a glycol, a polyol, a diamine, a polyamine, a hexamethoxymethylmelamine, a methylolacrylamide and any combination thereof.

6. The non-adhesive carboxylated latex dipped product according to claim 1, wherein the carboxyl-group blocking agent comprises a compound selected from the group consisting of glyoxals, polyamide compounds, polyamide polyurea compounds, polyamine polyurea compounds, polyamide polyurea glyoxyl condensation reaction product, polyamideamine compound, polyamideamine polyurea compounds, polyamideamine epihalohydrine condensation reaction products, polyamideamine formaldehyde condensation reaction products, polyamine epihalohydrine condensation reaction products, polyamine formaldehyde condensation reaction products, polyamide polyurea formaldehyde condensation reaction products, polyamine polyurea epihalohydrine condensation reaction products, polyamine polyurea formaldehyde condensation reaction products, polyamideamine polyurea epihalohydrine condensation reaction products, and polyamideamine polyurea formaldehyde condensation reaction products and combinations thereof.

7. The non-adhesive carboxylated latex dipped product according to claim 1, wherein the carboxyl-group blocking agent comprises a compound selected from the group consisting of monofunctional amines, monofunctional epoxy compounds, monofunctional isocyanates and monofunctional blocked isocyanates and combinations thereof.

8. The non-adhesive carboxylated latex dipped product according to claim 1, wherein the carboxyl-group blocking agent comprises a sizing agent.

9. The non-adhesive carboxylated latex product according to claim 1, wherein the carboxyl-group blocking agent comprises a surfactant.

10. A non-adhesive carboxylated latex dipped product, wherein the carboxylated latex is internally added with aluminate or aluminum hydroxide gel and a carboxyl-group blocking agent as defined in any one of claims 4 to 9.

11. The non-adhesive carboxylated latex dipped product according to any one of claims 1 to 10, wherein the carboxylated latex is crosslinked with aluminate or aluminium hydroxide gel.

12. The non-adhesive carboxylated latex dipped product according to any one of claims 1 to 11, wherein the carboxylated latex comprises an acrylonitrile-butadien rubber, a styrene-butadiene rubber, a chloroprene rubber or a methyl methacrylate-butadiene rubber.

13. The non-adhesive carboxylated latex dipped product according to any one of claims 1 to 12, wherein the dipped product is a fingerstall, a glove, a balloon or a condom.

14. The non-adhesive carboxylated latex dipped product according to claim 13 which is a fingerstall having a shape mechanically wound from the mouth before removed from the dipping former.

## Patentansprüche

1. Nicht-adhäsives carboxyliertes getauchtes Latexprodukt, bei dem eine Schicht auf einer oder beiden Oberflächen mit einem Carboxylgruppen-Blockierungsagens behandelt ist, wobei dem carboxylierten Latex intern Aluminat oder Aluminiumhydroxidgel zugegeben ist.

2. Nicht-adhäsives carboxyliertes getauchtes Latexprodukt gemäß Anspruch 1, worin das Carboxylgruppen-Blockierungsagens ein Metallionenvernetzungsmittel mit drei oder mehr Valenzen ist.

3. Nicht-adhäsives carboxyliertes getauchtes Latexprodukt gemäß Anspruch 2, worin das Metallionenvernetzungsmittel eine Aluminiumverbindung, Titanverbindung, Zirkoniumverbindung oder irgendeine Kombination davon ist.

4. Nicht-adhäsives carboxyliertes getauchtes Latexprodukt gemäß Anspruch 1, worin das Carboxylgruppen-Blockierungsagens ein organisches Vernetzungsmittel für Carboxylgruppen von carboxyliertem Latex ist.

5. Nicht-adhäsives carboxyliertes getauchtes Latexprodukt gemäß Anspruch 4, worin das organische Vernetzungsmittel eine Aziridinverbindung, eine Epoxyverbindung, ein blockiertes Isocyanat, eine Oxazolinverbindung, eine Carbodiimidverbindung, ein Melaminformaldehydharz, Harnstofformaldehydharze, Isocyanate, Phenolformaldehydharze, ein Glycol, ein Polyol, ein Diamin, ein Polyamin, ein Hexamethoxymethylmelamin, ein Methylolacrylamid und irgendeine Kombination davon umfaßt.

6. Nicht-adhäsives carboxyliertes getauchtes Latexprodukt gemäß Anspruch 1, worin das Carboxylgruppen-Blockierungsagens eine Verbindung umfaßt, die ausgewählt ist aus der Gruppe, die aus Glyoxalen, Polyamidverbindungen, Polyamidpolyharnstoff-Verbindungen, Polyaminpolyharnstoff-Verbindungen, Polyamidpolyharnstoffglyoxyl-Kondensationsreaktionsprodukt, Polyamidaminverbindung, Polyamidaminpolyharnstoff-Verbindungen, Polyamidaminepihalohydrin-Kondensationsreaktionsprodukten, Polyamidaminformaldehyd-Kondensationsreaktionsprodukten, Polyaminepihalohydrin-Kondensationsreaktionsprodukten, Polyaminformaldehyd-Kondensationsreaktionsprodukten, Polyamidpolyharnstofformaldehyd-Kondensationsreaktionsprodukten, Polyaminpolyharnstoffepihalohydrin-Kondensationsreaktionsprodukten, Polyaminpolyharnstofformaldehyd-Kondensationsreaktionsprodukten, Polyamidaminpolyharnstoffepihalohydrin-Kondensationsreaktionsprodukten und Polyamidaminpolyharnstofformaldehyd-Kondensationsreaktionsproduken und Kombinationen davon besteht.

7. Nicht-adhäsives carboxyliertes getauchtes Latexprodukt gemäß Anspruch 1, worin das Carboxylgruppen-Blockierungsagens eine Verbindung umfaßt, die aus der Gruppe ausgewählt ist, die aus monofunktionellen Aminen, monofunktionellen Epoxyverbindungen, monofunktionellen Isocyanaten und monofunktionellen blockierten Isocyanaten und Kombinationen davon besteht.

8. Nicht-adhäsives carboxyliertes getauchtes Latexprodukt gemäß Anspruch 1, worin das Carboxylgruppen-Blockierungsagens ein Leimungsmittel umfaßt.

9. Nicht-adhäsives carboxyliertes getauchtes Latexprodukt gemäß Anspruch 1, worin das Carboxylgruppen-Blockierungsagens ein Tensid umfaßt.

10. Nicht-adhäsives carboxyliertes getauchtes Latexprodukt, worin dem carboxylierten Latex intern Aluminat oder Aluminiumhydroxidgel und ein wie in einem der Ansprüche 4 bis 9 definiertes Carboxylgruppen-Blockierungsagens zugegeben ist.

11. Nicht-adhäsives carboxyliertes getauchtes Latexprodukt gemäß einem der Ansprüche 1 bis 10, worin der carboxylierte Latex mit Aluminat oder Aluminiumhydroxidgel vernetzt ist.

12. Nicht-adhäsives carboxyliertes getauchtes Latexprodukt gemäß einem der Ansprüche 1 bis 11, worin der carboxylierte Latex einen Acrylnitril-Butadien-Kautschuk, einen Styrol-Butadien-Kautschuk, einen Chloropren-Kautschuk oder einen Methylmethacrylat-Butadien-Kautschuk umfaßt.

13. Nicht-adhäsives carboxyliertes getauchtes Latexprodukt gemäß einem der Ansprüche 1 bis 12, worin das getauchte Produkt ein Fingerling, ein Handschuh, ein Ballon oder ein Kondom ist.

14. Nicht-adhäsives carboxyliertes getauchtes Latexprodukt gemäß Anspruch 13, das ein Fingerling mit einer Form ist, die mechanisch von der Öffnung gerollt wird, bevor er von der Tauchform entfernt wird.

## Revendications

1. Produit manufacturé par trempage en latex carboxylé non adhésif ayant une couche, sur une ou les deux surfaces, traitée avec un agent de blocage de groupe carboxyle, dans lequel le latex carboxylé est ajouté intérieurement avec un aluminate ou un gel d'hydroxyde d'aluminium.

2. Produit manufacturé par trempage en latex carboxylé non adhésif selon la revendication 1, dans lequel l'agent de blocage de groupe carboxyle est un agent de réticulation à ion métallique ayant trois valences ou plus.

3. Produit manufacturé par trempage en latex carboxylé non adhésif selon la revendication 2, dans lequel l'agent de réticulation à ion métallique est un composé d'aluminium, un composé de titane, un composé de zirconium ou n'importe quelle combinaison de ceux-ci.

4. Produit manufacturé par trempage en latex carboxylé non adhésif selon la revendication 1, dans lequel l'agent de blocage de groupe carboxyle est un agent de réticulation organique pour groupes carboxyle de latex carboxylé.

5. Produit manufacturé par trempage en latex carboxylé non adhésif selon la revendication 4, dans lequel l'agent de réticulation organique comprend un composé aziridine, un composé époxy, un isocyanate bloqué, un composé oxazoline, un composé carbodiimido, une résine mélamineformaldéhyde, des résines urée-formaldéhyde, des isocyanates, des résines phénolformaldéhyde, un glycol, un polyol, une diamine, une polyamine, une hexaméthoxyméthylmélamine, un méthylolacrylamide et n'importe quelle combinaison de ceux-ci.

6. Produit manufacturé par trempage en latex carboxylé non adhésif selon la revendication 1, dans lequel l'agent de blocage de groupe carboxyle comprend un composé choisi dans le groupe constitué de glyoxals, composés polyamide, composés polyamide polyurée, composés polyamine polyurée, produit de réaction de condensation polyamide polyurée glyoxyle, composé polyamideamine, composés polyamideamine polyurée, produits de réaction de condensation polyamideamine épihalohydrine, produits de réaction de condensation polyamideamine formaldéhyde, produits de réaction de condensation polyamine épihalohydrine, produits de réaction de condensation polyamine formaldéhyde, produits de réaction de condensation polyamide polyurée formaldéhyde, produits de réaction de condensation polyamine polyurée épihalohydrine, produits de réaction de condensation polyamine polyurée formaldéhyde, produits de réaction de condensation polyamide-amine polyurée épihalohydrine, et produits de réaction de condensation polyamideamine polyurée formaldéhyde et combinaisons de ceux-ci.

7. Produit manufacturé par trempage en latex carboxylé non adhésif selon la revendication 1, dans lequel l'agent de blocage de groupe carboxyle comprend un composé choisi dans le groupe constitué d'amines monofonctionnelles, composés époxy monofonctionnels, isocyanates monofonctionnels et isocyanates bloqués monofonctionnels et combinaisons de ceux-ci.

8. Produit manufacturé par trempage en latex carboxylé non adhésif selon la revendication 1, dans lequel l'agent de blocage de groupe carboxyle comprend un agent d'encollage.

9. Produit en latex carboxylé non adhésif selon la revendication 1, dans lequel l'agent de blocage de groupe carboxyle comprend un tensioactif.

10. Produit manufacturé par trempage en latex carboxylé non adhésif, dans lequel le latex carboxylé est ajouté intérieurement avec un gel d'aluminate ou d'hydroxyde d'aluminium et un agent de blocage de groupe carboxyle tel que défini dans l'une quelconque des revendications 4 à 9.

11. Produit manufacturé par trempage en latex carboxylé non adhésif selon l'une quelconque des revendications 1 à 10, dans lequel le latex carboxylé est réticulé avec un aluminate ou un gel d'hydroxyde d'aluminium.

12. Produit manufacturé par trempage en latex carboxylé non adhésif selon l'une quelconque des revendications 1 à 11, dans lequel le latex carboxylé comprend un caoutchouc acrylonitrile-butadiène, un caoutchouc styrène-butadiène, un caoutchouc chloroprène ou un caoutchouc méthacrylate de méthyle-butadiène.

13. Produit manufacturé par trempage en latex carboxylé non adhésif selon l'une quelconque des revendications 1 à 12, dans lequel le produit manufacturé par trempage est un doigtier, un gant, un ballon ou un condom.

14. Produit manufacturé par trempage en latex carboxylé non adhésif selon la revendication 13 qui est un doigtier ayant une forme enroulée mécaniquement à partir de l'ouverture avant retrait de la forme de trempage.
